(19) Europäisches Patentamt / European Patent Office / Office européen des brevets

(11) **EP 3 625 199 B1**

(12) **EUROPÄISCHE PATENTSCHRIFT**

(45) Veröffentlichungstag und Bekanntmachung des Hinweises auf die Patenterteilung:
**22.09.2021 Patentblatt 2021/38**

(21) Anmeldenummer: **18722580.0**

(22) Anmeldetag: **14.05.2018**

(51) Int Cl.:
***C07C 29/16*** *(2006.01)* ***C07C 31/12*** *(2006.01)*

(86) Internationale Anmeldenummer:
**PCT/EP2018/062328**

(87) Internationale Veröffentlichungsnummer:
**WO 2018/210720 (22.11.2018 Gazette 2018/47)**

(54) **HYDROFORMYLIERUNGSVERFAHREN DIENEND DER GROSSTECHNISCHEN HERSTELLUNG VON ALDEHYDEN UND/ODER ALKOHOLEN**

HYDROFORMYLATION METHOD SERVING FOR THE LARGE-SCALE PRODUCTION OF ALDEHYDES AND/OR ALCOHOLS

PROCÉDÉ D'HYDROFORMYLATION SERVANT DANS LA PRODUCTION INDUSTRIELLE D'ALCOOLS ET/OU D'ALDÉHYDES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.05.2017 EP 17171250**

(43) Veröffentlichungstag der Anmeldung:
**25.03.2020 Patentblatt 2020/13**

(73) Patentinhaber: **BASF SE**
**67056 Ludwigshafen am Rhein (DE)**

(72) Erfinder:
- **ZUEND, Stephan**
  **Fremont 94538 (US)**
- **PAPP, Rainer**
  **67056 Ludwigshafen (DE)**
- **BREITSCHEIDEL, Boris**
  **67056 Ludwigshafen (DE)**
- **LANGE DE OLIVEIRA, Armin**
  **69123 Heidelberg (DE)**

(74) Vertreter: **BASF IP Association**
**BASF SE**
**G-FLP-C006**
**67056 Ludwigshafen (DE)**

(56) Entgegenhaltungen:
**EP-A2- 1 219 584 US-A- 5 004 845**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 625 199 B1

Printed by Jouve, 75001 PARIS (FR)

## Beschreibung

Hydroformylierungsverfahren dienen der großtechnischen Herstellung von Aldehyden und/oder Alkoholen. Im Allgemeinen können Hydroformylierungsverfahren nach der Art des eingesetzten Katalysators in kobalt- oder rhodiumkatalysierte Hydroformylierungsverfahren untergliedert werden. Rhodiumkatalysierte Hydroformylierungsverfahren können weiter in Rhodium-Hochdruck- und Rhodium-Niederdruckverfahren untergliedert werden. Rhodium-Niederdruckverfahren können entweder als Kreisgasverfahren oder als Flüssigaustragsverfahren ausgestaltet sein. Industriell relevante Hydroformylierungsverfahren sind beispielsweise in Falbe, New Syntheses with Carbon Monoxide, Springer Berlin, 1980, Seite 162 bis 176 und in Ullmann's Encyclopedia of Industrial Chemistry, 2013 Wiley-VCH, Weinheim, doi10.1002/14356007.a8_312.pub2, Seite 1 bis 4 dargestellt.

Die Herstellung von Aldehyden durch Hydroformylierung erfolgt durch die Umsetzung von Olefinen in Gegenwart eines Hydroformylierungskatalysators, Kohlenstoffmonoxid und Wasserstoff. Die durch Hydroformylierung erhaltenen Aldehyde weisen ein Kohlenstoffatom mehr als die eingesetzten Olefine auf.

Aufgrund der reduktiven Bedingungen, die während der Hydroformylierung vorliegen, kann es sein, dass die Aldehyde zumindest teilweise zu den entsprechenden Alkoholen reduziert werden.

Je nach Art des Hydroformylierungsverfahrens und den gewählten Hydroformylierungsbedingungen kann Einfluss auf die Aldehydselektivität, auf die Hydrieraktivität und auf das n:iso-Verhältnis Einfluss genommen werden (siehe beispielsweise Ullmann's Encyclopedia of Industrial Chemistry, 2013, Wiley-VCH, Weinheim, doi:10.1002/14356007.a18_321.pub2).

Da Aldehyde reaktive Verbindungen darstellen, untergehen diese bereits während der Hydroformylierung unterschiedliche Reaktionen, die zur Bildung von Nebenprodukten, bzw. zur Bildung von komplexen Nebenproduktmischungen führen. Da diese Nebenprodukte meist eine höhere Molmasse und damit höhere Siedetemperaturen als die Hydroformylierungsprodukte aufweisen, werden diese Nebenprodukte auch als hochsiedende Nebenprodukte bezeichnet.

Hochsiedende Nebenprodukte bilden sich beispielsweise durch Aldolkondensation, Tischenko-Reaktion, Umesterungsreaktionen und/oder Disproportionierungsreaktionen als Dimere, Trimere und/oder Tetramere aus den Hydroformylierungsprodukten. Hochsiedende Nebenprodukte fallen dabei meist in der Form von Aldehyden, Estern, Acetalen und/oder Diolen an.

So ist in der US 4,148,830 beispielhaft die Bildung verschiedener hochsiedender Nebenprodukte aus den Hydroformylierungsprodukten der Propenhydroformylierung beschrieben. Gemäß der US 4,148,830 bilden sich die hochsiedenden Nebenprodukte beispielsweise durch Aldolkondensation, Tischenko-Reaktion, Umesterungsreaktionen und/oder Disproportionierungsreaktionen als Dimere, Trimere und/oder Tetramere in Form von Aldehyden, Estern, Acetalen und/oder Diolen.

Bei Hydroformylierungsprodukten handelt es sich in der Regel um die durch Hydroformylierung hergestellten Aldehyde, die ein Kohlenstoffatom mehr als die eingesetzten Olefine aufweisen. Aufgrund der reduktiven Bedingungen, die während der Hydrierung vorliegen kann es sich bei den Hydroformylierungsprodukten auch um Alkohole, die aus der Hydrierung vorstehender Aldehyde hervorgehen, oder um Mischungen von Aldehyden und Alkoholen handeln.

Die Abtrennung der hochsiedenden Nebenprodukte von den Hydroformylierungsprodukten erfolgt je nach Hydroformylierungsverfahren unterschiedlich. So kann die Abtrennung der hochsiedenden Nebenprodukte beispielsweise in einem Schritt, zusammen mit dem Hydroformylierungskatalysator, oder einem, der Abtrennung des Hydroformylierungskatalysators nachgeschaltetem, Verfahrensschritt erfolgen. Werden die hochsiedenden Nebenprodukte in einem der Abtrennung des Hydroformylierungskatalysators nachgeschaltetem Verfahrensschritt abgetrennt, erfolgt dies in der Regel im Rahmen der weiteren Aufarbeitung der Hydroformylierungsprodukte.

Im Falle der kobaltkatalysierten Hydroformylierung sind die hochsiedenden Nebenprodukte zusammen mit dem Hydroformylierungskatalysator und den Hydroformylierungsprodukten im Austrag der Hydroformylierung enthalten. Zur Abtrennung des Hydroformylierungskatalysators wird dieser in eine wasserlösliche Form überführt und anschließend per Flüssigextraktion entfernt. Eine vorteilhafte Verfahrensausgestaltung eines solchen Verfahrens ist beispielsweise in der WO 01/14297 beschrieben. Die hochsiedenden Nebenprodukte können anschließend bei der weiteren Aufarbeitung der Hydroformylierungsprodukte von diesen abgetrennt werden.

Im Kreisgasverfahren, das häufig zur Hydroformylierung von Propen eingesetzt wird, werden die Hydroformylierungsprodukte in der Gasphase am Kopf oder nahe am Kopf des Hydroformylierungsreaktors ausgetragen. Hochsiedende Nebenprodukte und der Hydroformylierungskatalysator bleiben dabei im Hydroformylierungsreaktor zurück. Der gasförmige Austrag wird kondensiert und die Hydroformylierungsprodukte werden von nicht umgesetztem Olefin, Wasserstoff, Kohlenstoffmonoxid und leichter siedenden Nebenprodukten, wie Propan, befreit. Die Hydroformylierungsprodukte werden anschließend der weiteren Aufarbeitung zugeführt. Nicht umgesetztes Olefin, Wasserstoff und Kohlenstoffmonoxid werden nach Abtrennung von Propan und Komprimierung in den Hydroformylierungsreaktor rückgeführt.

Im Flüssigaustragsverfahren, das häufig zur Hydroformylierung von Propen oder Butenen verwendet wird, können der Hydroformylierungskatalysator und die hochsiedende Nebenprodukte beispielswiese dadurch von den Hydroformylierungsprodukten abgetrennt werden, dass der Austrag der Hydroformylierung, der Hydroformylierungsprodukte, Hydroformylierungskatalysator und hochsiedende Nebenprodukte enthält, einer Destillation zugeführt wird, wobei der Hydroformylierungskatalysator und die hochsiedenden Nebenprodukte in der Sumpffraktion, die Hydroformylierungsprodukte in der Kopffraktion anfallen. Die in der Kopffraktion anfallenden Hydroformylierungsprodukte können dann der weiteren Aufarbeitung zugeführt werden. Es ist jedoch von Vorteil, wenn der Austrag der Hydroformylierung zuerst in einer oder mehreren Stufen entspannt wird, wobei der Austrag auf jeder Entspannungsstufe in eine flüssige und eine gasförmige Phase getrennt wird. Dadurch können beispielsweise leichter als die Hydroformylierungsprodukte siedende Nebenprodukte, nicht umgesetztes Olefin, Wasserstoff und/oder Kohlenstoffmonoxid aus dem Austrag der Hydroformylierung entfernt werden. Die gasförmige Phase und die flüssige Phase, die auf der letzten Entspannungsstufe anfallen, werden anschließend in einer Destillationskolonne im Gegenstrom geführt, wobei die Hydroformylierungsprodukte in der Kopffraktion und die hochsiedenden Nebenprodukte mit dem Hydroformylierungskatalysator in der Sumpffraktion anfallen. Die hochsiedenden Nebenprodukte und der Hydroformylierungskatalysator können in die Hydroformylierungsreaktion zurückgeführt, oder der Aufarbeitung des Hydroformylierungskatalysators zugeführt werden. Vorteilhafte Verfahrensausgestaltungen sind beispielsweise in der EP-A 0846095 oder EP-A 1255720 offenbart. Die in der Kopffraktion anfallenden Hydroformylierungsprodukte können anschließend der weiteren Aufarbeitung zugeführt werden.

Ist der Hydroformylierungskatalysator von den Hydroformylierungsprodukten weitgehend abgetrennt, gegebenenfalls zusammen mit den hochsiedenden Nebenprodukten, wird das so erhaltene Rohgemisch von Hydroformylierungsprodukten weiter aufgearbeitet. Die weitere Aufarbeitung umfasst im Allgemeinen eine destillative Aufreinigung und/oder Hydrierung.

Für ein Rohgemisch von Hydroformylierungsprodukte mit 4 oder 5 Kohlenstoffatomen kann die destillative Aufreinigung beispielsweise durch das in der EP-A 1 165 480 beschriebene Verfahren erfolgen. Für ein Rohgemisch von Hydroformylierungsprodukten mit 6 bis 13 Kohlenstoffatomen kann die destillative Aufreinigung beispielsweise durch das in der DE-A 199 14 260 beschriebene Verfahren erfolgen, auch wenn dieses Verfahren lediglich für Alkohole offenbart ist.

Durch Hydrierung der Hydroformylierungsprodukte wird ein Rohgemisch von Hydrierprodukten erhalten. Der Hydrierung kann ein Rohgemisch von Hydroformylierungsprodukten oder weitgehend reine Hydroformylierungsprodukte, wie sie beispielsweise nach der destillativen Aufreinigung eines Rohgemisches von Hydroformylierungsprodukten erhalten werden, zugeführt werden.

Bei Hydrierprodukten handelt es sich um Alkohole. Da die Hydrierprodukte aus der Hydrierung der Hydroformylierungsprodukte hervorgehen, weisen die Hydrierprodukte ein Kohlenstoffatom mehr als die zur Hydroformylierung eingesetzten Olefine auf.

Die Hydrierung der Hydroformylierungsprodukte, bzw. eines Rohgemisches von Hydroformylierungsprodukten erfolgt nach dem Fachmann bekannten Methoden. In der Regel erfolgt die Hydrierung in Gegenwart von Wasserstoff an einem Katalysator. Die Hydrierung stellt einen der Hydroformylierung nachgeschalteten Verfahrensschritt dar.

Als Katalysatoren werden im Allgemeinen heterogene Katalysatoren eingesetzt. Die Katalysatoren enthalten bevorzugt Metalle und/oder Metalloxide der VI. bis VIII. sowie der 1. Nebengruppe des Periodensystems der Elemente, insbesondere Chrom, Molybdän, Mangan, Rhenium, Eisen, Kobalt, Nickel und/oder Kupfer als katalytisch aktive Komponente. Die katalytisch aktive Komponente ist bevorzugt auf einem Trägermaterial abgeschieden. Das Trägermaterial enthält beispielsweise $Al_2O_3$, $SiO_2$, und/oder $TiO_2$. Solche Katalysatoren sind beispielsweise in der DE-A 3228881, DE-A2628987 und DE-A 2445303 beschreiben. Für die Hydrierung der Aldehyde wird die stöchiometrische Menge an Wasserstoff, oder ein Überschuss an Wasserstoff eingesetzt. In der Regel ist es bevorzugt, dass ein Überschuss an Wasserstoff eingesetzt wird. So kann beispielsweise ein Überschuss von 1.5 bis 20 Prozent über der stöchiometrisch zur Hydrierung der Aldehyde benötige Menge an Wasserstoff eingesetzt werden. Die Hydrierung der Aldehyde wird in der Regel bei einer Temperatur von 50 bis 200°C und bei einem Wasserstoffdruck von 25 bis 350 bar durchgeführt. Zur Vermeidung von Nebenreaktionen ist es bevorzugt, dass dem Hydrierzulauf gemäß DE-A 2628987 eine geringe Menge Wasser zugefügt wird. Es ist weiter bevorzugt, dass eine wässrige Lösung eines Alkalimetallhydroxids oder -carbonats entsprechend der Offenbarung der WO 01087809 dem Hydrierzulauf zugefügt wird.

Das so erhaltene Rohgemisch von Hydrierprodukten wird der weiteren Aufarbeitung zugeführt. Im Allgemeinen umfasst die weitere Aufarbeitung des Rohgemisches von Hydrierprodukten eine destillative Aufreinigung.

Für ein Rohgemisch von Hydrierprodukten mit 4 oder 5 Kohlenstoffatomen kann die destillative Aufreinigung beispielsweise analog dem in der EP-A 1 165 480 für Aldehyde beschriebenen Verfahren erfolgen. Für ein Rohgemisch von Hydrierprodukten mit 6 bis 13 Kohlenstoffatomen kann die destillative Aufreinigung beispielsweise analog dem in der DE-A 199 14 260 beschriebenen Verfahren erfolgen.

Aufgrund der thermischen Belastung der Hydroformylierungsprodukte oder Hydrierprodukte während der weiteren Aufarbeitung, im Speziellen während der Hydrierung und/oder der destillativen Aufreinigung, kann es auch hier zu Bildung von hochsiedenden Nebenprodukten kommen. Hochsiedende Nebenprodukte können sich beispielsweise analog der US 4,148,830 durch Aldolkondensation, Tischenko-Reaktion, Umesterungsreaktionen und/oder Disproportionierungsreaktionen der Aldehyde oder aus der Reaktion zwischen Aldehyd und Alkohol bilden.

Die hochsiedenden Nebenprodukte fallen bei der destillativen Aufreinigung des Rohgemisches der Hydroformylierungsprodukte oder des Rohgemisches der Hydrierprodukte in der Sumpffraktion an.

Da die hochsiedenden Nebenprodukte nur eine begrenzte wirtschaftliche Nutzbarkeit aufweisen, werden diese meist der thermischen Verwertung zugeführt.

Eine weitere Möglichkeit der Nutzbarmachung hochsiedender Nebenprodukte, beziehungsweise einer Sumpffraktion, in der diese Nebenprodukte enthalten sind, ist beispielsweise in der DE-B 2740216 offenbart. So beschreibt die DE-B 2740216 ein Verfahren zur Aufarbeitung von Destillationsrückständen der Propenhydroformylierung. Die Destillationsrückstände werden dabei unter Säurekatalyse in Gegenwart von n- oder iso-Butanol verestert, die erhaltenen Ester werden destillativ vom Katalysator und nicht verwertbaren Hochsiedern, wie Diolen, getrennt und anschließend an einem Kupferchromitkatalysator hydriert. Als Wertprodukte werden dabei n,iso-Butanol und 2-Ethylhexanol erhalten.

Auch wird in der US 3,501,537 ein Verfahren zur Aufarbeitung hochsiedender Nebenprodukte, die als Destillationsrückstände bei der Propen-Hydroformylierung anfallen offenbart. Die Destillationsrückstände werden dabei einer wiederholten Destillation unterzogen, um den Hydroformylierungskatalysator abzutrennen. Das dabei erhaltene Destillat wird anschließend direkt an einem Kupferchromitkatalysator hydriert oder in einem zweistufigen Verfahren, zuerst an einem Nickelkatalysator und anschließend an einem Kupferchromitkatalysator hydriert.

In der US 5,004,845 soll laut Beschreibung ein Verfahren zur Verfügung gestellt werden, bei welchem die Verluste an Alkohol, durch Nebenproduktbildung bei der katalytischen Hydrierung von $C_2$- bis $C_{10}$-Aldehyden, minimiert werden sollen. Hierzu scheint es sich als vorteilhaft zu erweisen, Ester, die sich als Nebenprodukte während der katalytischen Hydrierung gebildet haben, nach Abtrennung von den Alkoholen wieder in die katalytische Hydrierung rückzuführen, um damit das Gleichgewicht der während der Hydrierung ablaufenden Reaktionen günstig zu beeinflussen.

Aufgabe der vorliegenden Erfindung war es, ein Verfahren zur Verfügung zu stellen, mit diesem hochsiedende Nebenprodukte, die während der Hydroformylierung oder der weiteren Aufarbeitung eines Rohgemisches von Hydroformylierungsprodukten oder eines Rohgemisches von Hydrierprodukten gebildet werden, in Wertprodukte umzuwandeln. Im Rahmen der vorliegenden Erfindung sind Wertprodukte kurzkettige Alkohole, wie sie beispielsweise als Hydrierprodukte durch Hydrierung der Hydroformylierungsprodukte erhalten werden. Durch das erfindungsgemäße Verfahren soll es möglich sein, hochsiedende Nebenprodukte in Form von Acetalen und/oder Diolen in Wertprodukte umzuwandeln und somit die Ausbeute an Wertprodukten zu erhöhen.

Die Aufgabe wird gelöst durch ein Verfahren zur Herstellung von $C_4$- bis $C_{13}$-Monohydroxyverbindungen, vorzugsweise $C_4$- bis Cg-Monohydroxyverbindungen aus einer Sumpffraktion, die bei der Destillation eines Rohgemisches von $C_4$- bis $C_{13}$-Oxoaldehyden, vorzugsweise $C_4$- bis $C_9$-Oxoaldehyden aus der kobalt- oder rhodiumkatalysierten Hydroformylierung, oder bei der Destillation eines Rohgemisches von $C_4$- bis $C_{13}$-Oxoalkoholen, vorzugsweise $C_4$- bis $C_9$-Oxoalkoholen, anfällt, das dadurch gekennzeichnet ist,
dass die Sumpffraktion in Anwesenheit von Wasserstoff mit einem Katalysator enthaltend Kupferoxid (CuO) und Aluminiumoxid, bei einer Temperatur von 180°C bis 260°C und einem Druck von 150 bar bis 280 bar in Kontakt gebracht wird, das erhaltene rohe Hydrierprodukt einer Destillation unterzogen wird und dass die Menge an $C_4$- bis $C_{13}$-Monohydroxyverbindungen, vorzugsweise $C_4$- bis Cg-Monohydroxyverbindungen, die nach der Hydrierung im rohen Hydrierprodukt enthalten ist, größer ist als die Menge an $C_4$- bis $C_{13}$-Monohydroxyverbindungen, vorzugsweise $C_4$- bis Cg-Monohydroxyverbindungen, die sich aus der Hydrierung der in der Sumpffraktion enthaltenen Ester- und Aldehydverbindungen stöchiometrisch ergibt, einschließlich der in der Sumpffraktion vor der Hydrierung noch enthaltenen $C_4$- bis $C_{13}$-Monohydroxyverbindungen, vorzugsweise $C_4$- bis Cg-Monohydroxyverbindungen.

Handelt es sich bei den Hydroformylierungsprodukten um Aldehyde, werden die Hydroformylierungsprodukte auch als Oxoaldehyde bezeichnet. Wie bereits erwähnt weisen diese Aldehyde ein Kohlenstoffatom mehr als die zur Hydroformylierung eingesetzten Olefine auf.

Oxoalkohole werden durch Hydrierung von Oxoaldehyden erhalten. So können Oxoalkohole beispielsweise durch Hydroformylierung unter Verwendung von ligandenmodifizierten Kobaltcarbonylkomplexen als Hydroformylierungskatalysatoren (siehe beispielweise Falbe, New Syntheses with Carbon Monoxide, Springer Berlin, 1980, Seite 167 bis 168), oder durch separate Hydrierung der Hydroformylierungsprodukte, wenn es sich bei diesen um Oxoaldehyde handelt, erhalten werden.

Ein Rohgemisch von Oxoaldehyden, ist ein Gemisch das im Wesentlichen Oxoaldehyde enthält und aus diesem der Hydroformylierungskatalysator weitgehend abgetrennt ist. Ein Rohgemisch von Oxoaldehyden kann hochsiedende Nebenprodukte enthalten.

Bei einem Rohgemisch von $C_4$- bis $C_{13}$-Oxoaldehyden handelt es sich um ein Gemisch, das im Wesentlichen Oxoaldehyde mit gleicher Kohlenstoffanzahl enthält und aus diese der Hydroformylierungskatalysator weitgehend abgetrennt ist. Ein Rohgemisch von $C_4$- bis $C_{13}$-Oxoaldehyden kann hochsiedende Nebenprodukte enthalten.

So enthält ein Rohgemisch von $C_4$-Oxoaldehyden n-Butanal, iso-Butanal, oder eine Mischung dieser, wobei das Mischungsverhältnis von n-Butanal zu iso-Butanal von den Reaktionsbedingungen der Hydroformylierung abhängig ist. Ein Rohgemisch von $C_5$-Oxoaldehyden enthält beispielsweise, Valeraldehyd, Isovaleraldehyd, 2-Methylbutyraldehyd,

oder eine Mischung dieser, wobei das Mischungsverhältnis von den Reaktionsbedingungen und den Einsatzstoffen der Hydroformylierung abhängig ist (siehe beispielsweise EP-B1 1673332). Ein Rohgemisch von $C_6$-Oxoaldehyden enthält beispielsweise Capronaldehyde, 2-Methylpentan-1-al, 3-Methylpen-tan-1-al, 4-Methylpentan-1-al, 2,2-Dimethylbutan-1-al, 2,3-Dimethylbutan-1-al, 3,3-Dimethylbutan-1-al, 2-Ethylbutan-1-al, oder eine Mischung aus zwei, drei, vier oder mehr der genannten $C_6$-Oxoaldehyde. Ein Rohgemisch von $C_7$-Oxoaldehyden enthält beispielsweise n-Heptanal oder Isoheptanal. Ein Rohgemisch von $C_8$-Oxoaaldehyden enthält beispielsweise 2-Ethyl-2-hexenal, n-Octanal, oder Isooctanal. Ein Rohgemisch von $C_9$-Oxoaldehyden enthält beispielsweise n-Nonanal oder Isononanal. Ein Rohgemisch von $C_{10}$-Oxoaldehyden enthält beispielsweise 2-Propylheptanal, n-Decanal oder Isodecanal. Ein Rohgemisch von $C_{11}$-Oxoaldehyden enthält beispielsweise n-Undecanal oder Isoundecanal. Ein Rohgemisch von $C_{12}$-Oxoaldehyden enthält beispielsweise n-Dodecanal oder Isododecanal. Ein Rohgemisch von $C_{13}$-Oxoaldehyden enthält beispielsweise n-Tridecanal oder Isotridecanal.

Ein Rohgemisch von Oxoalkoholen, ist ein Gemisch das im Wesentlichen Oxoalkohole enthält und aus diesem der Hydroformylierungskatalysator weitgehend abgetrennt ist. Ein Rohgemisch von Oxoalkoholen kann hochsiedende Nebenprodukte enthalten. Ein Rohgemisch von Oxoalkoholen kann beispielsweise durch Hydrierung von reinen Oxoaldehyden, oder durch Hydrierung eines Rohgemisches von Oxoaldehyden erhalten werden.

Bei einem Rohgemisch von $C_4$- bis $C_{13}$-Oxoalkoholen handelt es sich um ein Gemisch, das im Wesentlich Oxoalkohole mit gleicher Kohlenstoffanzahl enthält und aus diesem der Hydroformylierungskatalysator weitgehend abgetrennt ist. Ein Rohgemisch von C4- bis C13-Oxoalkoholen kann hochsiedende Nebenprodukte enthalten.

So enthält ein Rohgemisch von $C_4$-Oxoalkoholen n-Butanol, iso-Butanol, oder eine Mischung dieser. Ein Rohgemisch von $C_5$-Oxoalkoholen enthält beispielsweise, n-Pentanol, 2-Methyl-1-butanol, 3-Methyl-1butanol, oder eine Mischung aus zwei, drei oder mehr der genannten $C_5$-Oxoalkohole. Ein Rohgemisch von $C_6$-Oxoalkoholen enthält beispielsweise n-Hexanol, 2-Methylpentan-1-ol, 3-Methylpentan-1-ol, 4-Methylpentan-1-ol, 2,3-Dimethylbutan-1-ol, 2-Ethylbutan-1-ol, oder eine Mischung aus zwei, drei oder mehr der genannten $C_6$-Oxoalkohole. Ein Rohgemisch von $C_7$-Oxoalkoholen enthält beispielsweise n-Heptanol oder Isoheptanol. Ein Rohgemisch von $C_8$-Oxoaalkoholenenthält beispielsweise 2-Ethylhexanol, n-Octanol, oder Isooctanol. Ein Rohgemisch von $C_9$-Oxoalkoholen enthält beispielsweise n-Nonanol oder Isononanol. Ein Rohgemisch von $C_{10}$-Oxoalkoholen enthält beispielsweise 2-Propylheptanol, n-Decanol oder Isodecanol. Ein Rohgemisch von $C_{11}$-Oxoalkoholen enthält beispielsweise n-Undecanol oder Isoundecanol. Ein Rohgemisch von $C_{12}$-Oxoalkoholen enthält beispielsweise n-Dodecanol oder Isododecanol. Ein Rohgemisch von $C_{13}$-Oxoalkoholen enthält beispielsweise n-Tridecanol oder Isotridecanol.

Die Herstellung von $C_4$- bis $C_{13}$- Oxoaldehyden und $C_4$- bis $C_{13}$-Oxoalkoholen ist dem Fachmann bekannt oder erschließt sich ihm aus seinem allgemeinen Fachwissen (siehe beispielsweise Ullmann's Encyclopedia of Industrial Chemistry, 2013 Wiley-VCH und Weinheim, doi:10.1002/14356007.a1_321.pub3, Ullmann's Encyclopedia of Industrial Chemistry, 2013 Wiley-VCH, Weinheim, doi:10.1002/14356007.a01_279.pub2).

Bei $C_4$- bis $C_{13}$-Monohydroxyverbindungen, die nach dem erfindungsgemäßen Verfahren erhalten werden, handelt es sich um Einzelverbindungen oder um Isomerenmischungen. Abhängig von der Kohlenstoffanzahl kann es möglich sein, die Isomerenmischungen destillativ in ihre Einzelverbindungen aufzutrennen. $C_4$-Monohydroxyverbindungen sind n-Butanol, iso-Butanol, oder eine Mischung dieser. $C_5$-Monohydroxyverbindungen sind n-Pentanol, 2-Methyl-1-butanol und 3-Methyl-1-butanol oder eine Mischung aus zwei, drei oder mehr der genannten $C_5$-Monohydroxyverbindungen. Isomerenmischungen von $C_4$- oder $C_5$-Monohydroxyverbindungen können in der Regel durch Destillation, beispielsweise durch das in der EP-A 1 165 480 offenbarte Verfahren, in ihre Einzelverbindungen aufgetrennt werden. Obgleich das Verfahren in der EP-A1 1 165 480 für Aldehyde offenbart ist, kann dieses auch zur Destillation von $C_4$- oder $C_5$-Monohydroxyverbindungen genutzt werden. So können Isomerenmischungen von $C_4$-Monohydroxyverbindungen durch Destillation beispielsweise in n-Butanol und iso-Butanol aufgetrennt werden. Monohydroxyverbindungen mit mehr als 5 Kohlenstoffatomen in der Kette werden bei der Destillation in der Regel als Isomerengemisch erhalten. Die Destillation von Monohydroxyverbindungen mit 6 bis 13 Kohlenstoffatomen kann beispielsweise nach dem in der D-A1 199 14 260 beschriebene Verfahren erfolgen. So werden $C_6$-Monohydroxyverbindungen in der Regel als n-Hexanol oder als Isomerenmischung erhalten. $C_7$-Monohydroxyverinbungen werden in der Regel als n-Heptanol oder als Isomerenmischung erhalten. $C_8$-Monohydroxyverbindungen werden in der Regel als 2-Ethylhexanol, n-Octanol oder Isooctanol erhalten. Cg-Monohydroxyverbindungen werden in der Regel als n-Nonanol oder Isononanol erhalten. $C_{10}$-Monohydroxyverbindungen werden in der Regel als 2-Propyl-heptanol, n-Decanol oder Isodecanol erhalten. $C_{11}$-Monohydroxyverbindungen werden in der Regel als n-Undecanol oder Isodecanol erhalten. $C_{12}$-Monohydroxyverbindungen werden in der Regel als n-Dodecanol oder Isododecanol erhalten. $C_{13}$-Monohydroxyverbindungen werden in der Regel als n-Tridecanol oder Isotridecanol erhalten.

Das erfindungsgemäße Verfahren eignet sich zur Herstellung von $C_4$- bis $C_{13}$-Monohydroxyverbindungen aus einer Sumpffraktion. Vorzugsweise eignet sich das erfindungsgemäße Verfahren zur Herstellung von $C_4$- bis Cg-Monohydroxyverbindungen, weiter bevorzugt zur Herstellung von $C_4$- oder Cg-Monohydroxyverbindungen und besonders bevorzugt zur Herstellung von $C_4$-Monohydroxyverbindungen.

Eine Sumpffraktion, die in dem erfindungsgemäßen Verfahren eingesetzt wird, fällt bei der Destillation eines

Rohgemisches von $C_4$- bis $C_{13}$-Oxoaldehyden oder bei der Destillation eines Rohgemisches von $C_4$- bis $C_{13}$-Oxoalkoholen an. Vorzugsweise fällt eine Sumpffraktion, die in dem erfindungsgemäßen Verfahren eingesetzt wird bei der Destillation eines Rohgemisches von $C_4$-bis $C_9$-Oxoaldehyden oder bei der Destillation eines Rohgemisches von $C_4$- bis $C_9$-Oxoalkoholen an. Weiter bevorzugt fällt eine Sumpffraktion, die in dem erfindungsgemäßen Verfahren eingesetzt wird bei der Destillation eines Rohgemisches von $C_4$- oder $C_9$-Oxoaldehyden oder bei der Destillation eines Rohgemisches von $C_4$- oder $C_9$-Oxoalkoholen an. Besonders bevorzugt fällt eine Sumpffraktion, die in dem erfindungsgemäßen Verfahren eingesetzt wird bei der Destillation eines Rohgemisches von $C_4$- Oxoaldehyden oder $C_4$-Oxoalkoholen an.

Es ist möglich eine Sumpffraktion, die bei der Destillation eines Rohgemisches von $C_4$- bis $C_{13}$-Oxoaldehyden anfällt mit einer Sumpffraktion, die bei der Destillation eines Rohgemisches von $C_4$- bis $C_{13}$-Oxoalholen anfällt zu kombiniert, bevor die kombinierte Sumpffraktion in dem erfindungsgemäßen Verfahren eingesetzt wird. Im Allgemeinen ist es zweckmäßig solche Sumpffraktionen zu kombinieren, die bei der Destillation von Oxoaldehyden und Oxoalkoholen mit gleicher Kohlenstoffanzahl anfallen. Dies hat den Vorteil, dass die destillative Aufreinigung, der durch das erfindungsgemäße Verfahren gewonnen Monohydroxyverbindungen, vereinfacht ist.

Neben hochsiedenden Nebenprodukten kann die Sumpffraktion Reste von $C_4$- bis $C_{13}$-Oxoaldehyden und/oder $C_4$- bis $C_{13}$-Oxoalkoholen enthalten. So kann die Sumpffraktion beispielsweise Reste von $C_4$- bis $C_9$-Oxoaldehyden und/oder $C_4$- bis $C_9$-Oxoalkoholen enthalten. Weiter kann die Sumpffraktion beispielsweise Reste von $C_4$- oder $C_9$-Oxoaldehyden und/oder $C_4$- oder Cg-Oxoalkoholen enthalten. Die Sumpffraktion kann auch Reste von $C_4$- Oxoaldehyden und/oder $C_4$-Oxoalkoholen enthalten.

Die Destillation eines Rohgemisches von $C_4$- bis $C_{13}$-Oxoaldehyden oder eines Rohgemisches von $C_4$- bis $C_{13}$-Oxoalkoholen erfolgt kontinuierlich oder diskontinuierlich. Die Destillation wird als normale Destillation oder als Rektifikation ausgeführt. Die Destillation wird in einer oder mehreren Destillationskolonnen ausgeführt. Generell ist es vorteilhaft die Destillation als Rektifikation auszuführen, wobei eine oder mehrere Rektifikationskolonnen verwendet werden. Wird die Rektifikation in mehreren Rektifikationskolonnen ausgeführt ist es vorteilhaft, mehrere hintereinandergeschaltete Rektifikationskolonnen, beispielsweise 2, 3 oder 4, zu verwenden.

Als Kolonnen für die Destillation, beziehungsweise Rektifikation, eignen sich beispielsweise Bodenkolonnen, wie Ventilbodenkolonne. Im Allgemeinen sind Kolonnen mit Packungen bevorzugt. Bei Packungen handelt es sich beispielsweise um regellose Schüttungen oder um geordnete Packungen. Im Allgemeinen sind geordnete Packungen bevorzugt. Die Zahl der Trennstufen kann der Fachmann aufgrund seines Fachwissens und durch wenige Routineversuche an die gewünschte Trennwirkung anpassen.

Vorteilhafte Verfahrensausgestaltungen zur Destillation eines Rohgemisches von $C_4$- bis $C_{13}$-Oxoaldehyden oder eines Rohgemisches von $C_4$- bis $C_{13}$-Oxoalkoholen sind beispielsweise in der EP-B1 1 165 480 oder in der DE-A1 199 14 260 offenbart. Die in der EP-B1 1 165 480 und DE-A1 199 14 260 beschriebenen Verfahren eignen sich sowohl für die Destillation eines Rohgemisches von Oxoaldehyden als auch für die Destillation eine Rohgemisches von Oxoalkoholen

Die Sumpffraktion kann einer weiteren Destillation, Strippung und/oder einer Extraktion unterzogen werden, bevor diese in dem erfindungsgemäßen Verfahren eingesetzt wird. Durch die genannten Verfahrensmaßnahmen können beispielsweise anorganische Verbindungen, Reste des Hydroformylierungskatalysators und/oder andere Nebenprodukte sowie phosphorhaltige Verbindungen abgereichert werden. Dadurch kann eine Schädigung des Katalysators in dem erfindungsgemäßen Verfahren, beispielsweise durch Verkokung und/oder Verstopfung der Katalysatorporen bzw. Vergiftung reduziert werden, womit die Standzeit erhöht wird.

Wird die Sumpffraktion einer weiteren Destillation unterzogen, bevor diese in dem erfindungsgemäßen Verfahren eingesetzt wird erfolgt dies bei erhöhter Temperatur und vermindertem Druck. Eine solche Destillation erfolgt beispielsweise bei einer Temperatur von 70 bis 220 °C und bei einem Druck von 10 bis 500 mbar. Das bei einer solchen Destillation erhaltene Destillat, das unter anderem hochsiedende Nebenprodukte enthält, wird dann in dem erfindungsgemäßen Verfahren eingesetzt.

Wird eine Sumpffraktion destilliert, bevor diese in dem erfindungsgemäßen Verfahren eingesetzt wird, kann die Destillation in einer oder mehreren Kolonnen erfolgen. Eine solche Destillation kann als Destillation an einem Fallfilmverdampfer oder als Flash-Destillation erfolgen. Eine solche Destillation kann kontinuierlich oder diskontinuierlich erfolgen.

So kann die Sumpffraktion, bevor diese in dem erfindungsgemäßen Verfahren eingesetzt wird, einer kontinuierlichen oder diskontinuierlichen Flash-Destillation bei einer Temperatur von 70 bis 220 °C und bei einem Druck von 10 bis 500 mbar unterzogen werden.

Auch kann die Sumpffraktion, bevor diese in dem erfindungsgemäßen Verfahren eingesetzt wird, einer kontinuierlichen oder diskontinuierlichen Destillation bei einer Temperatur von 70 bis 220 °C und bei einem Druck von 10 bis 500 mbar an einem Fallfilmverdampfer unterzogen werden.

Bei einer Strippung wird die Sumpffraktion mit einem Strippmedium im Gleich- oder Gegenstrom in Kontakt gebracht. Bei einem Strippmedium handelt es sich im Allgemeinen um ein Gas wie Luft, Stickstoff und/oder Wasserdampf. Eine Strippung kann nach bekannten Methoden des Standes der Technik ausgeführt werden.

Eine Extraktion wird im Allgemeinen als Flüssig-Flüssig-Extraktion ausgeführt. Eine Extraktion kann kontinuierlich oder diskontinuierlich erfolgen. Eine Extraktion kann einen oder mehrere Extraktionsschritte, beispielsweise 2, 3 oder 4, umfassen. In den jeweiligen Extraktionsschritten kann das Extraktionsmedium gleich oder unterschiedlich sein. Im Allgemeinen ist es bevorzugt, dass in einem Extraktionsschritt Wasser als Extraktionsmedium verwendet wird. Bevorzugt handelt es sich hierbei um demineralisiertes Wasser.

Die genaue Zusammensetzung der Sumpffraktion unterscheidet sich in der Regel stark, je nach der Stelle, an der die Sumpffraktion anfällt. So hängt die Zusammensetzung der Sumpffraktion beispielsweise von den Ausgangsstoffen der Hydroformylierung oder Hydrierung ab.

Wird beispielsweise weitgehend reiner n-Butyraldehyd hydriert, kann die Sumpffraktion, die dann bei der Destillation des Rohgemisches von $C_4$-Oxoalkoholen anfällt, unter anderem folgende Verbindungen enthalten:

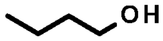
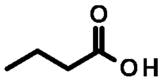

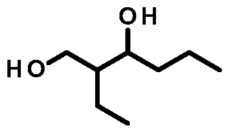
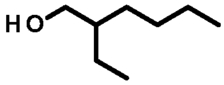
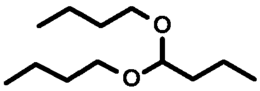

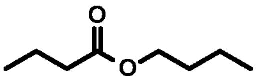
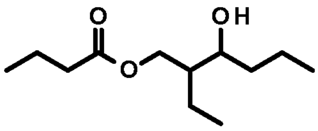
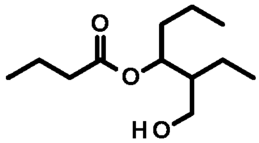

Wird beispielsweise ein Gemisch aus weitgehend reinem n-Butyraldehyd und iso-Butyraldehyd hydriert, kann die Sumpffraktion, die dann bei der Destillation des Rohgemisches von $C_4$-Oxoalkoholen anfällt, unter anderem folgende Verbindungen enthalten:

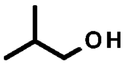
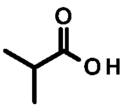
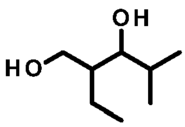
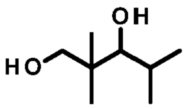
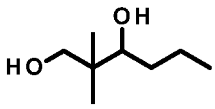

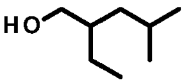
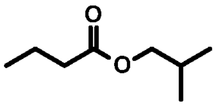
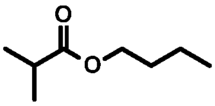
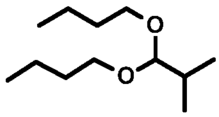

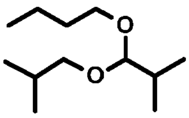
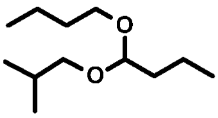
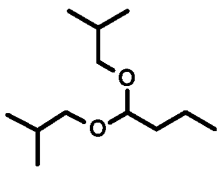
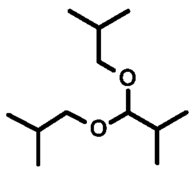

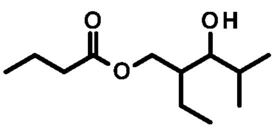
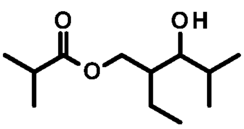
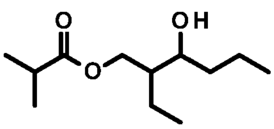
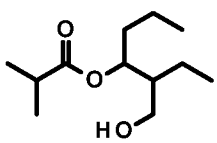

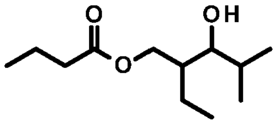 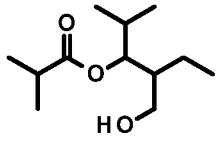 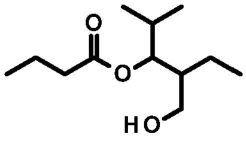 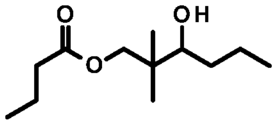

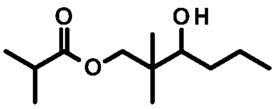 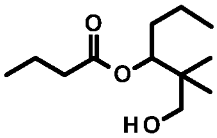 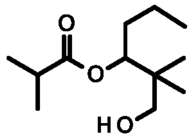 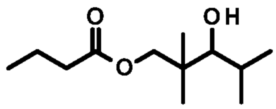

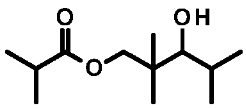 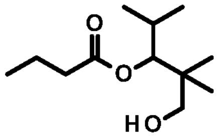 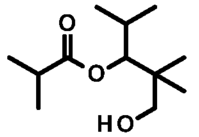 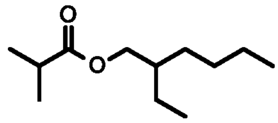

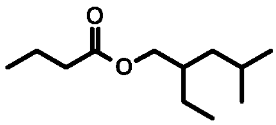 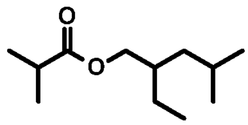

Gegenstand der Erfindung ist demnach auch ein Verfahren zur Herstellung von $C_4$-Monohydroxyverbindungen aus einer Sumpffraktion, die bei der Destillation eines Rohgemisches von $C_4$-Oxoaldehyden aus der rhodiumkatalysierten Hydroformylierung, oder bei der Destillation eines Rohgemisches von $C_4$-Oxoalkoholen anfällt, das dadurch gekennzeichnet ist,
dass die Sumpffraktion in Anwesenheit von Wasserstoff mit einem Katalysator enthaltend Kupferoxid (CuO) und Aluminiumoxid, bei einer Temperatur von 150°C bis 300°C und einem Druck von 20 bar bis 300 bar in Kontakt gebracht wird, das erhaltene rohe Hydrierprodukt einer Destillation unterzogen wird und dass die Menge an $C_4$-Monohydroxyverbindungen die nach der Hydrierung im rohen Hydrierprodukt enthalten ist, größer ist als die Menge an $C_4$-Monohydroxyverbindungen, die sich aus der Hydrierung der in der Sumpffraktion enthaltenen Ester- und Aldehydverbindungen stöchiometrisch ergibt, einschließlich der in der Sumpffraktion vor der Hydrierung noch enthaltenen $C_4$-Monohydroxyverbindungen.

Nach dem erfindungsgemäßen Verfahren wird die Sumpffraktion in Anwesenheit von Wasserstoff mit einem Katalysator in Kontakt gebracht. Bei dem Katalysator handelt es sich um einen heterogenen Katalysator. Der Katalysator enthält Kupferoxid (CuO) und Aluminiumoxid. Es ist bevorzugt, dass der Katalysator neben Kupferoxid (CuO) und Aluminiumoxid eines oder mehrere Oxide des Mangans, Lanthans, Wolframs, Molybdäns, Titans, Zinks oder Zirkoniums enthält. Es ist weiter bevorzugt, dass der Katalysator neben Kupferoxid (CuO) und Aluminiumoxid eines oder mehrere Oxide des Mangans, Lanthans oder Zinks enthält. Es ist besonders bevorzugt, dass der Katalysator neben Kupferoxid (CuO) und Aluminiumoxid eines oder mehrere Oxide des Lanthans oder Zinks enthält.

In der Regel enthält der Katalysator 40 bis 95 Gewichtsprozent Kupferoxid (CuO) und 5 bis 60 Gewichtsprozent Aluminiumoxid. Der Katalysator kann zusätzlich bis zu 30 Gewichtsprozent an einem oder mehreren Oxiden des Mangans, Lanthans, Wolframs, Molybdäns, Titans, Zinks oder Zirkoniums enthalten. Enthält der Katalysator neben Kupferoxid und Aluminiumoxid zusätzlich ein oder mehrere Oxide des Mangans, Lanthans, Wolframs, Molybdän, Titans, Zinks oder Zirkoniums addiert sich die Summe der Gewichtsanteile auf 90 bis 100 Gewichtsprozent. Die Gewichtsprozentangaben beziehen sich auf das Gesamtgewicht des im Katalysator enthaltenen oxidischen Materials nach der Calcinierung. Der Anteil an oxidischem Material im Katalysator beträgt mindestens 80 Gewichtsprozent bezogen auf das Gesamtgewicht des Katalysators nach Calcinierung. So beträgt der Anteil an oxidischem Material im Katalysator bezogen auf das Gesamtgewicht des Katalysators nach Calcinierung bevorzugt 80 bis 100 Gewichtsprozent, weiter bevorzugt 90 bis 100 Gewichtsprozent.

Es ist bevorzugt, dass der Katalysator 40 bis 80 Gewichtsprozent Kupferoxid (CuO), 5 bis 60 Gewichtsprozent Aluminiumoxid und 0 bis 30 Gewichtsprozent Manganoxid (MnO), Lanthanoxid oder Zinkoxid enthält. Die Gewichtsangaben beziehen sich jeweils auf das Gesamtgewicht des im Katalysator enthaltenen oxidischen Materials nach Calcinierung und addieren sich auf einen Wert von 90 bis 100 Gewichtsprozent. Der Anteil an oxidischem Material im Katalysator beträgt mindestens 80 Gewichtsprozent bezogen auf das Gesamtgewicht des Katalysators nach Calcinierung. So beträgt der Anteil an oxidischem Material im Katalysator bezogen auf das Gesamtgewicht des Katalysators nach Calcinierung bevorzugt 80 bis 100 Gewichtsprozent, weiter bevorzugt 90 bis 100 Gewichtsprozent.

Ein für das erfindungsgemäße Verfahren bevorzugter Katalysator, der Kupferoxid (CuO), Aluminiumoxid und Zinkoxid enthält, ist in der EP-B1 0901982 offenbart. Die chemische Zusammensetzung eines solchen Katalysators, wie er in der EP-B1 0901982 offenbart ist, kann in weiten Grenzen variieren. Vorzugsweise beträgt das Atomverhältnis Cu:Zn 1:5 bis 5:1, besonders bevorzugt 1:1 bis 4:1, insbesondere 2:1 bis 3:1. Das Atomverhältnis (Cu+Zn): Al beträgt vorzugsweise 99:1 bis 70:30, besonders bevorzugt 95:5 bis 80:20. Speziell bevorzugt ist ein Verhältnis Cu:Zn:Al von etwa 65:25:10. Dies entspricht einer chemischen Zusammensetzung von 67 Gewichtsprozent CuO, 26,4 Gewichtsprozent ZnO und 6,6 Gewichtsprozent $Al_2O_3$ im fertigen Katalysator (also bezogen auf das Gesamtgewicht des Katalysators nach Calcinierung).

Neben den Elementen Kupfer, Zink und Aluminium kann der Katalysator auch ein oder mehrere Elemente ausgewählt aus den Platinmetlallen, den Gruppen IV, V, XI und den Lanthaniden des Periodensystems der Elemente enthalten. Bevorzugte Beispiele sind Pd, Pt, Rh, Ru, Os, Au, Zr, Ti, V, Nb, Ta sowie die Lanthaniden.

Zur Herstellung eines solchen Cu-Zn-Al-Katalysators wird beispielsweise zunächst eine Zn-Al-Mischoxidverbindung erzeugt, die anschließend durch Calcinierung in eine, zumindest teilweise säureunlösliche, Phase umgewandelt wird, diese Phase in saurer Lösung suspendiert wird und anschließend in Gegenwart dieser Phase eine Cu-Zn-Mischoxidverbindung erzeugt wird. Unter Mischoxidverbindungen werden Oxid-, Hydroxid-, Carbonat-, und Hydroxycarbonatverbindungen verstanden. Insgesamt wird ein Mischoxid aus Zn-Al-Mischoxid und Cu-Zn-Mischoxid erhalten. Dieses wird dann bei Temperaturen von 20 bis 400°C getrocknet und bei Temperaturen von 200 bis 800°C calciniert. Genaue Ausführungsformen zur Herstellung eines solchen Katalysators sind in der EP-B1 0901982 offenbart.

Ebenfalls für das erfindungsgemäße Verfahren bevorzugt ist ein Katalysator, der Kupferoxid (CuO) Aluminiumoxid und Lanthanoxid enthält. Ein solcher Katalysator ist beispielsweise in der WO 2004/085356 offenbart. Ein solcher Katalysator enthält 55 bis 75 Gewichtsprozent Kupferoxid (CuO), 20 bis 30 Gewichtsprozent Aluminiumoxid und 3 bis 15 Gewichtsprozent Lanthanoxid, als oxidisches Material. Die Gewichtsangaben beziehen sich jeweils auf das Gesamtgewicht des im Katalysator enthaltenen oxidischen Materials nach Calcinierung und addieren sich auf einen Wert von 90 bis 100 Gewichtsprozent. Der Anteil an oxidischem Material im Katalysator beträgt mindestens 80 Gewichtsprozent bezogen auf das Gesamtgewicht des Katalysators nach Calcinierung. So beträgt der Anteil an oxidischem Material im Katalysator bezogen auf das Gesamtgewicht des Katalysators nach Calcinierung bevorzugt 80 bis 100 Gewichtsprozent, weiter bevorzugt 90 bis 100 Gewichtsprozent.

Einem solchen Katalysator kann nach Calcinierung zusätzlich metallisches Kupferpulver, Kupferblättchen, Zementpulver, Graphit oder ein Gemisch davon mit einem Anteil von 0,5 bis 20 Gewichtsprozent, bezogen auf das Gesamtgewicht des im Katalysator enthaltenen oxidischen Materials zugemischt werden. Wird dem Katalysator metallisches Kupferpulver, Kupferblättchen Zementpulver, Graphit oder ein Gemisch davon zugemischt wird die so erhaltene Mischung bei einer Temperatur von 270°C bis 400°C erneut calciniert. Zementpulver wird dabei nicht als oxidisches Material betrachtet. Bevorzugt weist ein solcher Katalysator eine chemische Zusammensetzung von 55 bis 60 Gewichtsprozent CuO, 25 bis 30 Gewichtsprozent $Al_2O_3$, 8 bis 10 Gewichtsprozent $La_2O_3$ und 3 bis 6 Gewichtsprozent metallisches Kupfer auf. Die Gewichtsangaben beziehen sich jeweils auf das Gesamtgewicht des Katalysators nach Calcinierung und addieren sich auf einen Wert von 90 bis 100 Gewichtsprozent.

Die Herstellung solcher Katalysatoren ist ebenfalls in der WO 2004/085356 offenbart. So wird beispielsweise eine wässrige Lösung von Kupfernitrat, Aluminiumnitrat und Lanthannitrat mit einer wässrigen Natriumcarbonatlösung unter pH-Kontrolle gemischt. Das Reaktionsgemisch wird anschließend nitratfrei gewaschen, getrocknet und bei einer Temperatur von 300°C calciniert. Das nach der Kalzinierung erhaltene Gemisch wird dann mit Graphit kompaktiert und das Kompaktat mit metallischem Kupfer und Graphit gemischt und zu Tabletten verpresst. Die erhaltenen Tabletten werden schließlich bei 350°C calciniert (2h).

Ein ebenfalls für das erfindungsgemäße Verfahren bevorzugter Katalysator enthält 55 bis 70 Gewichtsprozent Kupferoxid (CuO), 20 bis 35 Gewichtsprozent Aluminiumoxid und 3 bis 15 Gewichtsprozent Manganoxid (MnO). Die Gewichtsangaben beziehen sich jeweils auf das Gesamtgewicht des im Katalysator enthaltenen oxidischen Materials nach Calcinierung und addieren sich auf einen Wert von 90 bis 100 Gewichtsprozent. Der Anteil an oxidischem Material im Katalysator beträgt mindestens 80 Gewichtsprozent bezogen auf das Gesamtgewicht des Katalysators nach Calcinierung. So beträgt der Anteil an oxidischem Material im Katalysator bezogen auf das Gesamtgewicht des Katalysators nach Calcinierung bevorzugt 80 bis 100 Gewichtsprozent, weiter bevorzugt 90 bis 100 Gewichtsprozent. Besonders bevorzugt weist ein solcher Katalysator eine chemische Zusammensetzung von 58 bis 62 Gewichtsprozent Kupferoxid (CuO), 8 bis 12 Gewichtsprozent Manganoxid (MnO) und 28 bis 32 Gewichtsprozent Aluminiumoxid auf. Die Gewichtsangaben beziehen sich jeweils auf das Gesamtgewicht des im Katalysator enthaltenen oxidischen Materials nach Calcinierung und addieren sich auf einen Wert von 90 bis 100 Gewichtsprozent. Der Anteil an oxidischem Material im Katalysator beträgt bevorzugt mindestens 80 Gewichtsprozent bezogen auf das Gesamtgewicht des Katalysators nach Calcinierung. So beträgt der Anteil an oxidischem Material im Katalysator bezogen auf das Gesamtgewicht des Katalysators nach Calcinierung bevorzugt 80 bis 100 Gewichtsprozent, weiter bevorzugt 90 bis 100 Gewichtsprozent.

Die Herstellung manganhaltiger Katalysatoren ist beispielsweise in der WO 97/34694 offenbart. Zur Herstellung wird beispielsweise eine wässrige Kupfernitratlösung ($Cu(NO_3)_2$), eine wässrige Mangannitratlösung und eine wässrige

Natriumaluminatlösung ($Na_2Al_2O_4$) unter pH-Kontrolle in eine wässrige Natriumcarbonatlösung ($Na_2CO_3$) eingemischt. Der sich bildende Niederschlag wird abfiltriert, gewaschen und bei einer Temperatur von bis zu 150°C getrocknet. Das trockene Produkt wird anschließend bei einer Temperatur von 300 bis 1000°C calciniert. Alternativ wird beispielsweise eine wässrige Kupfernitratlösung ($Cu(NO_3)_2$) und eine wässrige Natriumaluminatlösung ($Na_2Al_2O_4$) unter pH-Kontrolle in eine wässrige Natriumcarbonatlösung ($Na_2CO_3$) eingemischt. Der sich bildende Niederschlag wird abfiltriert, gewaschen und bei einer Temperatur von bis zu 150°C getrocknet. Das trockene Produkt wird anschließend bei einer Temperatur von 300 bis 1000°C calciniert. Das kalzinierte Produkt wird anschließend mit einer wässrigen Manganlösung, beispielsweise Manganchloridlösung, imprägniert und wiederholt bei einer Temperatur von 300 bis 1000°C calciniert. Alle Calzinierungsschritte werden in Gegenwart von Sauerstoff durchgeführt.

Ein weiterer für das erfindungsgemäße Verfahren bevorzugter Katalysator enthält 40 bis 60 Gewichtsprozent Kupferoxid (CuO) und 60 bis 40 Gewichtsprozent Aluminiumoxid. Die Gewichtsangaben beziehen sich jeweils auf das Gesamtgewicht des im Katalysator enthaltenen oxidischen Materials nach Calcinierung und addieren sich auf einen Wert von 90 bis 100 Gewichtsprozent. Der Anteil an oxidischem Material im Katalysator beträgt bevorzugt mindestens 80 Gewichtsprozent bezogen auf das Gesamtgewicht des Katalysators nach Calcinierung. So beträgt der Anteil an oxidischem Material im Katalysator bezogen auf das Gesamtgewicht des Katalysators nach Calcinierung bevorzugt 80 bis 100 Gewichtsprozent, weiter bevorzugt 90 bis 100 Gewichtsprozent.

Ein solcher Katalysator kann beispielsweise analog der WO 97/34694 hergestellt werden.

Um die Sumpffraktion in Anwesenheit von Wasserstoff mit einem Katalysator in Kontakt zu bringen, werden die Sumpffraktion und Wasserstoff in einen Reaktor eingeleitet, in diesem sich der Katalysator befindet.

Die Sumpffraktion wird in der flüssigen Phase in den Reaktor eingeleitet. Es ist bevorzugt, dass die Sumpffraktion mit einem flüssigen Inertmedium verdünnt ist. Dies hat beispielsweise den Vorteil, dass die Viskosität der Sumpffraktion erniedrigt werden kann, wodurch diese leichter förderbar ist. Auch kann die Verdünnung der Sumpffraktion mit einem flüssigen Inertmedium dazu dienen, die Wärmeabführung während der Reaktion günstig zu beeinflussen.

Ein Inertmedium geht unter den Reaktionsbedingungen keine Reaktion mit den Edukten, Produkten und/oder Katalysator ein. Beispielsweise handelt es sich bei einem flüssigen Inertmedium um einen langkettigen Kohlenwasserstoff. Der Anteil an einem Inertmedium beträgt bevorzugt 10 bis 80 Gewichtsprozent, weiter bevorzugt 20 bis 70 Gewichtsprozent und besonders bevorzugt 25 bis 65 Gewichtsprozent, bezogen auf das Gesamtgewicht von Sumpffraktion und Inertmedium.

Es ist bevorzugt, dass die Sumpffraktion so mit dem Katalysator in Kontakt gebracht, dass sich eine Gesamtflüssigkeitsbelastung des Katalysators von 0,01 bis 5 $g_{Sumpffraktion}/(ml_{Katalysator}*h)$ ergibt. Es ist weiter bevorzugt, dass sich eine Gesamtflüssigkeitsbelastung von 0,3 bis 5 $g_{Sumpffraktion}/(ml_{Katalysator}*h)$ ergibt. Wird die Sumpffraktion mit einem flüssigen Inertmedium verdünnt, ist darauf zu achten, dass sich die Gesamtflüssigkeitsbelastung des Katalysators in dem zuvor definierten Rahmen bewegt.

Wasserstoff wird bevorzugt möglichst rein in den Reaktor eingeleitet. Reiner Wasserstoff hat eine Reinheit von mindestens 95 Gewichtsprozent. Verunreinigungen im Wasserstoff können beispielsweise Methan und/oder Stickstoff sein.

Wasserstoff wird mindestens in der stöchiometrischen Menge, bezogen auf die zu reduzierenden Verbindungen, die in der Sumpffraktion enthalten sind, in den Reaktor eingeleitet. So kann die Menge an Wasserstoff die in den Reaktor eingeleitet wird 100 bis 300 Prozent der stöchiometrischen Menge, bezogen auf die zu reduzierenden Verbindungen, die in der Sumpffraktion enthalten sind, betragen. Es ist bevorzugt, dass die Menge an Wasserstoff 105 bis 200 Prozent, weiter bevorzugt 110 bis 180 Prozent und besonders bevorzugt 120 bis 160 Prozent beträgt. Die Menge an Wasserstoff kann beispielsweise 101, 102, 108, 112, 115, 118, 122, 125, 130, 135, 140, 145, 150 oder 155 Prozent der stöchiometrischen Menge, bezogen auf die zu reduzierenden Verbindungen, die in der Sumpffraktion enthalten sind, betragen.

Es ist zweckmäßig den Katalysator vor Verwendung in dem erfindungsgemäßen Verfahren in eine aktive Form zu überführen. Der Katalysator wird hierzu beispielsweise in Gegenwart eines wasserstoffhaltigen Gases nach einem Temperaturprogramm reduziert. Bei einem wassersstoffhaltigen Gas handelt es sich um ein Gas, das 1 bis 99 Prozent Wasserstoff und 99 bis 1 Prozent eines gasförmigen Inertmediums wie Stickstoff, Argon und/oder Helium enthält. Um beispielsweise eine bessere Wärmeabführung während der Aktivierung des Katalysators zu gewährleisten, ist es weiterhin zweckmäßig, die Aktivierung in Gegenwart eines flüssigen Inertmediums durchzuführen. Bei einem flüssigen Inertmedium handelt es sich beispielsweise um einen langkettigen Kohlenwasserstoff oder um eine Mischung von langkettigen Kohlenwasserstoffen.

Als Reaktor für das erfindungsgemäße Verfahren eigenen sich prinzipiell alle Reaktoren in denen Hydrierreaktionen, vorzugsweise Hydrierreaktionen in der Flüssigphase, durchgeführt werden können und die für eine Hydrierung bei einer Temperatur von 150 bis 300°C und einen Druck von 20 bis 300 bar geeignet sind. So können beispielsweise Rührkesselreaktoren, Rührkesselkaskaden, Kammerreaktoren, Blasensäulen, Schlaufenreaktoren, Rieselfilmreaktoren, Bodenkolonnen, Dünnschichtreaktoren, Strahldüsenreaktoren, pulsierende Kolonnen, Festbettreaktoren, Festbett-Röhrenreaktoren, Festbettreaktoren mit innenliegenden oder außenliegenden Wärmetauschern, Wirbelschichtreaktoren, mehrstufige Wirbelschichtreaktoren, Wanderbettreaktoren, oder eine beliebige Kombination dieser für das erfindungsgemäße Verfahren genutzt werden. Mehrere gleiche oder verschiedene Reaktortypen können in Reihe oder parallel geschaltet sein. Reaktoren, die sich für das erfindungsgemäße Verfahren eignen sind beispielsweise in J. Falbe, Katalysatoren, Tenside und Mineralöladditive, Georg Thieme Verlag, Stuttgart, 1978, Seite 30 bis 37 beschrieben.

Es sind solche Reaktortypen bevorzugt, bei denen der Katalysator in einem Festbett oder in einer Schüttung angeordnet ist. Es ist möglich, dass in einem Reaktor mehrere Katalysator Festbetten oder Schüttungen in Reihe angeordnet sind. Der Reaktor untergliedert sich dabei in Zonen mit Katalysator (Reaktionszonen) und Zonen ohne Katalysator, die sich gegenseitig abwechseln. Die Anzahl der Reaktionszonen in einem Reaktor kann 2 bis 10 betragen.

Die Sumpffraktion und Wasserstoff werden über eine oder mehrere Zuleitungen in den Reaktor eingeleitet. Es ist bevorzugt, dass Sumpffraktion und Wasserstoff über eine oder mehrere separate Zuleitungen in den Reaktor eingeleitet werden. Auch wenn nicht bevorzugt ist es generell auch möglich, dass die Sumpffraktion und Wasserstoff vorvermischt werden und über eine oder mehrere gemeinsame Zuleitungen in den Reaktor eingeleitet werden.

Es ist bevorzugt, dass die Sumpffraktion in Rieselfahrweise oder in Sumpffahrweise in den Reaktor eingeleitet wird. Die Sumpffraktion wird dabei im Gleich- oder Gegenstrom mit Wasserstoff in den Reaktor eingeleitet, wobei ein Einleiten im Gleichstrom bevorzugt ist.

Wird für das erfindungsgemäße Verfahren ein Reaktor mit mehreren Reaktionszonen verwendet, kann Wasserstoff auch zwischen den Reaktionszonen in den Reaktor eingeleitet und dort mit der Sumpffraktion in Kontakt gebracht werden. Dies hat beispielsweise den Vorteil, dass die Temperaturentwicklung während der Reaktion besser kontrolliert werden kann. Eine vorteilhafte Verfahrensausgestaltung ist beispielsweise in der EP-A1 0 073 129 beschrieben.

Das erfindungsgemäße Verfahren wird kontinuierlich oder diskontinuierlich ausgeführt, wobei eine kontinuierliche Ausführung bevorzugt ist.

Das erfindungsgemäße Verfahren kann adiabatisch oder nicht adiabatisch ausgeführt werden. Wird das erfindungsgemäße Verfahren adiabatisch ausgeführt, ist es von Vorteil, wenn ein Reaktor mit mehreren Reaktionszonen verwendet wird. Während die Reaktion in den einzelnen Reaktionszonen weitgehend adiabatisch abläuft, können zwischen den Reaktionszonen kältere Edukte in Form von Wasserstoff oder frischer Sumpffraktion zugeleitet werden. Dies ermöglicht eine weitgehend adiabatische Reaktionsführung mit verbesserter Temperaturkontrolle. Eine vorteilhafte Ausgestaltungsform für ein derartiges Verfahren ist beispielsweise in der EP-A1 0 073 129 offenbart.

Im Rahmen der vorliegenden Erfindung werden die Sumpffraktion, Wasserstoff und der Katalysator im Allgemeinen bei einer Temperatur von 150 bis 300°C, bevorzugt 180 bis 260°C in Kontakt gebracht.

Die Sumpffraktion, Wasserstoff und der Katalysator werden im Allgemeinen bei einem Druck von 20 bis 300 bar, bevorzugt 100 bis 300 bar und weiter bevorzugt 150 bis 280 bar in Kontakt gebracht.

Durch das erfindungsgemäße Verfahren wird eine Reaktionsmischung erhalten, die auch als rohes Hydrierprodukt bezeichnet wird. Das rohe Hydrierprodukt aus dem erfindungsgemäßen Verfahren, bzw. aus der erfindungsgemäßen Hydrierung, kann neben $C_4$- bis $C_{13}$-Monohydroxyverbindungen verschiedene Nebenprodukte, wie hochsiedende Nebenprodukte, enthalten.

Die Menge an $C_4$- bis $C_{13}$-Monohydroxyverbindungen, die in dem rohen Hydrierprodukt enthalten ist, ist größer als die stöchiometrische Menge an $C_4$- bis $C_{13}$-Monohydroxyverbindungen, die sich aus der Hydrierung der in der Sumpffraktion enthaltenen Ester- und Aldehydverbindungen ergibt, einschließlich der in der Sumpffraktion vor der Hydrierung schon enthaltenen $C_4$- bis $C_{13}$-Monohydroxyverbindungen.

Die Berechnung der Menge an $C_4$- bis $C_{13}$-Monohydroxyverbindungen, die nach dem erfindungsgemäßen Verfahren erhalten werden kann, soll im Folgenden beispielhaft für die $C_4$-Monohydroxyverbindung n-Butanol dargestellt werden. Die Sumpffraktion wird in diesem Fall bei der Destillation eines Rohgemisches von n-Butanol gewonnen.

In einem ersten Schritt wird die Sumpffraktion, die in dem erfindungsgemäßen Verfahren eingesetzt wird, dahingehend analysiert, ob die darin enthaltenen Komponenten formal auf $C_4$- oder $C_8$-Bestandteile zurückgeführt werden können. Die Analyse erfolgt beispielsweise per GC, nachdem die Retentionszeiten der einzelnen Verbindungen durch GC/MS aufgeklärt wurden. Die in der Sumpffraktion enthaltenen Komponenten können dann wie folgt beispielhaft eingeteilt werden:

n-Butanol: 100% $C_4$
n-Butyl-Butyrat: 100% $C_4$
n-Butyraldehyd-n,n-Dibutylacetal: 100% $C_4$
$C_8$-Diole: 100% $C_8$
2-Ethylhexan-1-ol: 100% $C_8$
$C_{12}$-Ester: 33% $C_4$ und 67% $C_8$

Durch Summierung der Komponenten kann ein $C_4$-Anteil, ein $C_8$-Anteil und ein Anteil an nicht aufgeschlüsselten Komponenten in der Sumpffraktion bestimmt werden. Die Summe an n-Butanol, die im rohen Hydrierprodukt erhalten wird, ist die $C_4$-Gesamtausbeute.

Unter der Annahme, dass der Umsatz von den $C_4$-Komponenten in der Sumpffraktion zu n-Butanol vollständig

abläuft, kann anhand des Wertes der $C_4$-Gesamtausbeute im rohen Hydrierprodukt, die Umsetzung der $C_8$-Komponenten berechnet werden.

$C_4$-Gesamtausbeute - $C_4$-Anteil in der Sumpffraktion - Anteil der nicht aufgeschlüsselten Komponenten = Ausbeute an $C_4$-Komponenten durch Umsetzung von $C_3$-Komponenten.

Durch diese Berechnung kann festgestellt werden, wie viele $C_8$-Komponenten zu n-Butanol umgesetzt werden. Die Bestimmung der Zusammensetzung des rohen Hydrierprodukts kann ebenfalls mittels GC/MS, bzw. GC erfolgen.

Das rohe Hydrierprodukt aus dem erfindungsgemäßen Verfahren wird einer Destillation unterzogen. Bei einer Destillation handelt es sich um eine normale Destillation oder Rektifikation. Die Destillation erfolgt kontinuierlich oder diskontinuierlich. Die Destillation wird in einer oder mehreren Destillationskolonnen ausgeführt. Generell ist es vorteilhaft die Destillation als Rektifikation auszuführen, wobei eine oder mehrere Rektifikationskolonnen verwendet werden. Wird die Rektifikation in mehreren Rektifikationskolonnen ausgeführt ist es vorteilhaft, mehrere hintereinandergeschaltete Rektifikationskolonnen, beispielsweise 2, 3 oder 4, zu verwenden.

Als Kolonnen für die Destillation, beziehungsweise Rektifikation, eignen sich beispielsweise Bodenkolonnen, wie Ventilbodenkolonne. Im Allgemeinen sind Kolonnen mit Packungen bevorzugt. Bei Packungen handelt es sich beispielsweise um regellose Schüttungen oder um geordnete Packungen handeln. Im Allgemeinen sind geordnete Packungen bevorzugt. Die Zahl der Trennstufen kann der Fachmann aufgrund seines Fachwissens und durch wenige Routineversuche an die gewünschte Trennwirkung anpassen.

Vorteilhafte Verfahrensausgestaltungen zur Destillation eines rohen Hydrierprodukts aus dem erfindungsgemäßen Verfahren sind in der EP-B1 1 165 480 oder in der DE-A 199 14 260 offenbart. Obwohl das in der EP-B1 offenbarte Verfahren zur Destillation von Aldehyden beschrieben wird, kann dieses auch zur Destillation von Monohydroxyverbindungen genutzt werden.

Enthält das rohe Hydrierprodukt aus dem erfindungsgemäßen Verfahren noch hochsiedende Nebenprodukte kann es zweckmäßig sein, dieses zumindest teilweise in das erfindungsgemäße Verfahren zurückzuführen bevor dieses einer Destillation unterzogen wird. Wird das rohe Hydrierprodukt zumindest teilweise in das erfindungsgemäße Verfahren zurückgeführt, wird das rohe Hydrierprodukt aufgeteilt, wobei ein Teil dem Verfahren entnommen wird, während der andere Teil in das erfindungsgemäße Verfahren zurückgeführt wird. Das Verhältnis zwischen dem Teil, der dem Verfahren entnommen wird, zu dem Teil, der in das erfindungsgemäße Verfahren zurückgeführt wird, beträgt bevorzugt 1:1 bis 20:1. Es ist bevorzugt, dass das Verhältnis 2:1 bis 10:1 beträgt. Der Teil der in das erfindungsgemäße Verfahren zurückgeführt wird kann mit frischer Sumpffraktion vermischt werden oder separat dem erfindungsgemäßen Verfahren zugeleitet werden. Das Verhältnis zwischen frischer Sumpffraktion zu dem Teil der in das erfindungsgemäße Verfahren zurückgeführt wird beträgt bevorzugt 1:1 bis 1:20. Es ist weiter bevorzugt, dass das Verhältnis 1:4 bis 1:15 beträgt. Es ist besonders bevorzugt, dass das Verhältnis 1:4 zu 1:10 beträgt.

Die Rückführung von zumindest einem Teil des rohen Hydrierprodukts in das erfindungsgemäße Verfahren erlaubt einen wirtschaftlichen Betrieb des Verfahrens auch bei niedrigeren Umsätzen. Durch niedrigere Umsätze kann die Verweilzeit und damit die thermische Belastung der Sumpffraktion reduziert werden. Diese neigt daher weniger zur Bildung von unerwünschten Nebenprodukten, die zur Verkokung des Katalysators führen können.

Wird ein Teil in das erfindungsgemäße Verfahren zurückgeführt ist darauf zu achten, dass sich die Gesamtflüssigkeitsbelastung des Katalysators in dem zuvor definierten Rahmen bewegt.

Der Teil der dem erfindungsgemäßen Verfahren entnommen wird, wird einer Destillation, bevorzugt einer Rektifikation unterzogen.

Ohne an diese Theorie gebunden zu sein, wurde herausgefunden, dass durch das erfindungsgemäße Verfahren neben Estern- und Aldehydverbindungen auch Diole und Acetale in Monohydroxyverbindungen umgewandelt werden können. Das erfindungsgemäße Verfahren kann demnach dazu genutzt werden, um aus einer Sumpffraktion, die hochsiedende Nebenprodukte enthält, Hydrierprodukte zu gewinnen, wodurch eine höhere Gesamtausbeute an Hydrierprodukten erreicht werden kann. Bei Hydrierprodukten handelt es sich wie eingangs definiert um Alkohole, die aus der Hydrierung der Hydroformylierungsprodukte hervorgehen und damit ein Kohlenstoffatom mehr als die zur Hydroformylierung eingesetzten Olefine aufweisen. Da das erfindungsgemäße Verfahren in üblichen Reaktoren, die für großtechnische Hydrierungen geeignet sind, durchgeführt werden kann, lässt es sich zudem einfach in bestehenden Apparaturen ausführen.

Nachdem überraschenderweise herausgefunden wurde, dass durch das erfindungsgemäße Verfahren auch Diole zu Monohydroxyverbindungen umgewandelt werden können, ist Gegenstand der vorliegenden Erfindung auch ein Verfahren zur Herstellung von $C_4$-Monohydroxyverbindungen durch Hydrogenolyse von $C_8$-Diolen, das dadurch gekennzeichnet ist, dass $C_8$-Diole in Anwesenheit von Wasserstoff mit einem Katalysator enthaltend Kupferoxid (CuO) und Aluminiumoxid, bei einer Temperatur von 150 bis 300°C und einem Druck von 20 bis 300 bar in Kontakt gebracht werden, die $C_8$-Diolezumindest teilweise in $C_4$-Monohydroxyverbindungen umgewandelt werden und das erhaltene Reaktionsgemisch einer Destillation unterzogen wird.

$C_8$-Diolekönnen unabhängig von ihrer Herkunftsquelle in dem erfindungsgemäßen Verfahren eingesetzt werden. Bei $C_8$-Diolen handelt es sich in der Regel um ein Isomerengemisch. Es ist auch möglich $C_8$-Diolein Form von

chemischen Einzelverbindungen in dem erfindungsgemäßen Verfahren einzusetzen.

$C_8$-Diolewerden bevorzugt als Bestandteil von Mischungen in dem erfindungsgemäßen Verfahren eingesetzt. So können $C_8$-Diole beispielsweise in einer Sumpffraktion enthalten sein, die bei einer Destillation eines Rohgemisches von $C_4$-Oxoaldehyden aus dem Austrag der rhodiumkatalysierten Hydroformylierung oder bei einer Destillation eines Rohgemisches von $C_4$-Oxoalkoholen aus dem Austrag der Hydrierung eines Rohgemisches von $C_4$-Oxoaldehyden anfällt.

$C_8$-Diole sind geradkettig oder verzweigt. Es ist bevorzugt, dass $C_8$-Diole eine chemische Struktur aufweisen, bei der durch Hydrogenolyse und damit verbundenem C-C Bindungsbruch zwei n-Butanole, zwei iso-Butanole oder ein n-Butanol und ein iso-Butanol entstehen. $C_8$-Diole, die diese Spezifikation erfüllen sind beispielsweise im obigen dargestellt. Ein $C_8$-Diole, das durch Hydrogenolyse und damit verbundenem C-C Bindungsbruch in zwei n-Butanole überführt werden kann, ist beispielsweise 2-Ethylhexan-1,3-diol.

Durch das erfindungsgemäße Verfahren zur Hydrogenolyse von $C_8$-Diolen werden als $C_4$-Monohydroxyverbindungen n-Butanol, iso-Butanol oder eine Mischung dieser erhalten. Die Art der $C_4$-Monohydroxyverbindungen, die durch das erfindungsgemäße Verfahren erhalten werden, hängt von der Struktur der eingesetzten $C_8$-Diole ab.

Nach dem erfindungsgemäßen Verfahren werden $C_8$-Diole in Anwesenheit von Wasserstoff mit einem Katalysator in Kontakt gebracht. Bei dem Katalysator handelt es sich um einen heterogenen Katalysator.

Der Katalysator enthält Kupferoxid (CuO) und Aluminiumoxid. Es ist bevorzugt, dass der Katalysator neben Kupferoxid (CuO) und Aluminiumoxid eines oder mehrere Oxide des Mangans, Lanthans, Wolframs, Molybdäns, Titans, Zinks oder Zirkoniums enthält. Es ist weiter bevorzugt, dass der Katalysator neben Kupferoxid (CuO) und Aluminiumoxid eines oder mehrere Oxide des Mangans, Lanthans oder Zinks enthält. Es ist besonders bevorzugt, dass der Katalysator neben Kupferoxid (CuO) und Aluminiumoxid eines oder mehrere Oxide des Lanthans oder Zinks enthält.

In der Regel enthält der Katalysator 40 bis 95 Gewichtsprozent Kupferoxid (CuO) und 5 bis 60 Gewichtsprozent Aluminiumoxid. Der Katalysator kann zusätzlich bis zu 30 Gewichtsprozent an einem oder mehreren Oxiden des Mangans, Lanthans, Wolframs, Molybdäns, Titans, Zinks oder Zirkoniums enthalten. Enthält der Katalysator neben Kupferoxid und Aluminiumoxid zusätzlich ein oder mehrere Oxide des Mangans, Lanthans, Wolframs, Molybdän, Titans, Zinks oder Zirkoniums addiert sich die Summe der Gewichtsanteile auf 90 bis 100 Gewichtsprozent. Die Gewichtsprozentangaben beziehen sich auf das Gesamtgewicht des im Katalysator enthaltenen oxidischen Materials nach der Calcinierung. Der Anteil an oxidischem Material im Katalysator beträgt mindestens 80 Gewichtsprozent bezogen auf das Gesamtgewicht des Katalysators nach Calcinierung. So beträgt der Anteil an oxidischem Material im Katalysator bezogen auf das Gesamtgewicht des Katalysators nach Calcinierung bevorzugt 80 bis 100 Gewichtsprozent, weiter bevorzugt 90 bis 100 Gewichtsprozent.

Es ist bevorzugt, dass der Katalysator 40 bis 80 Gewichtsprozent Kupferoxid (CuO), 5 bis 60 Gewichtsprozent Aluminiumoxid und 0 bis 30 Gewichtsprozent Manganoxid (MnO), Lanthanoxid oder Zinkoxid enthält. Die Gewichtsangaben beziehen sich jeweils auf das Gesamtgewicht des im Katalysator enthaltenen oxidischen Materials nach Calcinierung und addieren sich auf einen Wert von 90 bis 100 Gewichtsprozent. Der Anteil an oxidischem Material im Katalysator beträgt mindestens 80 Gewichtsprozent bezogen auf das Gesamtgewicht des Katalysators nach Calcinierung. So beträgt der Anteil an oxidischem Material im Katalysator bezogen auf das Gesamtgewicht des Katalysators nach Calcinierung bevorzugt 80 bis 100 Gewichtsprozent, weiter bevorzugt 90 bis 100 Gewichtsprozent.

Ein für das erfindungsgemäße Verfahren bevorzugter Katalysator, der Kupferoxid (CuO), Aluminiumoxid und Zinkoxid enthält, ist in der EP-B1 0901982 offenbart. Die chemische Zusammensetzung eines solchen Katalysators, wie er in der EP-B1 0901982 offenbart ist, kann in weiten Grenzen variieren. Vorzugsweise beträgt das Atomverhältnis Cu:Zn 1:5 bis 5:1, besonders bevorzugt 1:1 bis 4:1, insbesondere 2:1 bis 3:1. Das Atomverhältnis (Cu+Zn): Al beträgt vorzugsweise 99:1 bis 70:30, besonders bevorzugt 95:5 bis 80:20. Speziell bevorzugt ist ein Verhältnis Cu:Zn:Al von etwa 65:25:10. Dies entspricht einer chemischen Zusammensetzung von 67 Gewichtsprozent CuO, 26,4 Gewichtsprozent ZnO und 6,6 Gewichtsprozent $Al_2O_3$ im fertigen Katalysator (also bezogen auf das Gesamtgewicht des Katalysators nach Calcinierung).

Neben den Elementen Kupfer, Zink und Aluminium kann der Katalysator auch ein oder mehrere Elemente ausgewählt aus den Platinmetlallen, den Gruppen IV, V, XI und den Lanthaniden des Periodensystems der Elemente enthalten. Bevorzugte Beispiele sind Pd, Pt, Rh, Ru, Os, Au, Zr, Ti, V, Nb, Ta sowie die Lanthaniden.

Zur Herstellung eines solchen Cu-Zn-Al-Katalysators wird beispielsweise zunächst eine Zn-Al-Mischoxidverbindung erzeugt, die anschließend durch Calcinierung in eine, zumindest teilweise säureunlösliche, Phase umgewandelt wird, diese Phase in saurer Lösung suspendiert wird und anschließend in Gegenwart dieser Phase eine Cu-Zn-Mischoxidverbindung erzeugt wird. Unter Mischoxidverbindungen werden Oxid-, Hydroxid-, Carbonat-, und Hydroxycarbonatverbindungen verstanden. Insgesamt wird ein Mischoxid aus Zn-Al-Mischoxid und Cu-Zn-Mischoxid erhalten. Dieses wird dann bei Temperaturen von 20 bis 400°C getrocknet und bei Temperaturen von 200 bis 800°C calciniert. Genaue Ausführungsformen zur Herstellung eines solchen Katalysators sind in der EP-B1 0901982 offenbart.

Ebenfalls für das erfindungsgemäße Verfahren bevorzugt ist ein Katalysator, der Kupferoxid (CuO) Aluminiumoxid und Lanthanoxid enthält. Ein solcher Katalysator ist beispielsweise in der WO 2004/085356 offenbart. Ein solcher

Katalysator enthält 55 bis 75 Gewichtsprozent Kupferoxid (CuO), 20 bis 30 Gewichtsprozent Aluminiumoxid und 3 bis 15 Gewichtsprozent Lanthanoxid, als oxidisches Material. Die Gewichtsangaben beziehen sich jeweils auf das Gesamtgewicht des im Katalysator enthaltenen oxidischen Materials nach Calcinierung und addieren sich auf einen Wert von 90 bis 100 Gewichtsprozent. Der Anteil an oxidischem Material im Katalysator beträgt mindestens 80 Gewichtsprozent bezogen auf das Gesamtgewicht des Katalysators nach Calcinierung. So beträgt der Anteil an oxidischem Material im Katalysator bezogen auf das Gesamtgewicht des Katalysators nach Calcinierung bevorzugt 80 bis 100 Gewichtsprozent, weiter bevorzugt 90 bis 100 Gewichtsprozent.

Einem solchen Katalysator kann nach Calcinierung zusätzlich metallisches Kupferpulver, Kupferblättchen, Zementpulver, Graphit oder ein Gemisch davon mit einem Anteil von 0,5 bis 20 Gewichtsprozent, bezogen auf das Gesamtgewicht des im Katalysator enthaltenen oxidischen Materials zugemischt werden. Wird dem Katalysator metallisches Kupferpulver, Kupferblättchen Zementpulver, Graphit oder ein Gemisch davon zugemischt wird die so erhaltene Mischung bei einer Temperatur von 270°C bis 400°C erneut calciniert. Zementpulver wird dabei nicht als oxidisches Material betrachtet. Bevorzugt weist ein solcher Katalysator eine chemische Zusammensetzung von 55 bis 60 Gewichtsprozent CuO, 25 bis 30 Gewichtsprozent $Al_2O_3$, 8 bis 10 Gewichtsprozent $La_2O_3$ und 3 bis 6 Gewichtsprozent metallisches Kupfer auf. Die Gewichtsangaben beziehen sich jeweils auf das Gesamtgewicht des Katalysators nach Calcinierung und addieren sich auf einen Wert von 90 bis 100 Gewichtsprozent.

Die Herstellung solcher Katalysatoren ist ebenfalls in der WO 2004/085356 offenbart. So wird beispielsweise eine wässrige Lösung von Kupfernitrat, Aluminiumnitrat und Lanthannitrat mit einer wässrigen Natriumcarbonatlösung unter pH-Kontrolle gemischt. Das Reaktionsgemisch wird anschließend nitratfrei gewaschen, getrocknet und bei einer Temperatur von 300°C calciniert.

Das nach der Kalzinierung erhaltene Gemisch wird dann mit Graphit kompaktiert und das Kompaktat mit metallischem Kupfer und Graphit gemischt und zu Tabletten verpresst. Die erhaltenen Tabletten werden schließlich bei 350°C calciniert (2h).

Ein ebenfalls für das erfindungsgemäße Verfahren bevorzugter Katalysator enthält 55 bis 70 Gewichtsprozent Kupferoxid (CuO), 20 bis 35 Gewichtsprozent Aluminiumoxid und 3 bis 15 Gewichtsprozent Manganoxid (MnO). Die Gewichtsangaben beziehen sich jeweils auf das Gesamtgewicht des im Katalysator enthaltenen oxidischen Materials nach Calcinierung und addieren sich auf einen Wert von 90 bis 100 Gewichtsprozent. Der Anteil an oxidischem Material im Katalysator beträgt mindestens 80 Gewichtsprozent bezogen auf das Gesamtgewicht des Katalysators nach Calcinierung. So beträgt der Anteil an oxidischem Material im Katalysator bezogen auf das Gesamtgewicht des Katalysators nach Calcinierung bevorzugt 80 bis 100 Gewichtsprozent, weiter bevorzugt 90 bis 100 Gewichtsprozent. Besonders bevorzugt weist ein solcher Katalysator eine chemische Zusammensetzung von 58 bis 62 Gewichtsprozent Kupferoxid (CuO), 8 bis 12 Gewichtsprozent Manganoxid (MnO) und 28 bis 32 Gewichtsprozent Aluminiumoxid auf. Die Gewichtsangaben beziehen sich jeweils auf das Gesamtgewicht des im Katalysator enthaltenen oxidischen Materials nach Calcinierung und addieren sich auf einen Wert von 90 bis 100 Gewichtsprozent. Der Anteil an oxidischem Material im Katalysator beträgt bevorzugt mindestens 80 Gewichtsprozent bezogen auf das Gesamtgewicht des Katalysators nach Calcinierung. So beträgt der Anteil an oxidischem Material im Katalysator bezogen auf das Gesamtgewicht des Katalysators nach Calcinierung bevorzugt 80 bis 100 Gewichtsprozent, weiter bevorzugt 90 bis 100 Gewichtsprozent.

Die Herstellung manganhaltiger Katalysatoren ist beispielsweise in der WO 97/34694 offenbart. Zur Herstellung wird beispielsweise eine wässrige Kupfernitratlösung ($Cu(NO_3)_2$), eine wässrige Mangannitratlösung und eine wässrige Natriumaluminatlösung ($Na_2Al_2O_4$) unter pH-Kontrolle in eine wässrige Natriumcarbonatlösung ($Na_2CO_3$) eingemischt. Der sich bildende Niederschlag wird abfiltriert, gewaschen und bei einer Temperatur von bis zu 150°C getrocknet. Das trockene Produkt wird anschließend bei einer Temperatur von 300 bis 1000°C calciniert. Alternativ wird beispielsweise eine wässrige Kupfernitratlösung ($Cu(NO_3)_2$) und eine wässrige Natriumaluminatlösung ($Na_2Al_2O_4$) unter pH-Kontrolle in eine wässrige Natriumcarbonatlösung ($Na_2CO_3$) eingemischt. Der sich bildende Niederschlag wird abfiltriert, gewaschen und bei einer Temperatur von bis zu 150°C getrocknet. Das trockene Produkt wird anschließend bei einer Temperatur von 300 bis 1000°C calciniert. Das kalzinierte Produkt wird anschließend mit einer wässrigen Manganlösung, beispielsweise Manganchloridlösung, imprägniert und wiederholt bei einer Temperatur von 300 bis 1000°C calciniert. Alle Calzinierungsschritte werden in Gegenwart von Sauerstoff durchgeführt.

Ein weiterer für das erfindungsgemäße Verfahren bevorzugter Katalysator enthält 40 bis 60 Gewichtsprozent Kupferoxid (CuO) und 60 bis 40 Gewichtsprozent Aluminiumoxid. Die Gewichtsangaben beziehen sich jeweils auf das Gesamtgewicht des im Katalysator enthaltenen oxidischen Materials nach Calcinierung und addieren sich auf einen Wert von 90 bis 100 Gewichtsprozent. Der Anteil an oxidischem Material im Katalysator beträgt bevorzugt mindestens 80 Gewichtsprozent bezogen auf das Gesamtgewicht des Katalysators nach Calcinierung. So beträgt der Anteil an oxidischem Material im Katalysator bezogen auf das Gesamtgewicht des Katalysators nach Calcinierung bevorzugt 80 bis 100 Gewichtsprozent, weiter bevorzugt 90 bis 100 Gewichtsprozent.

Ein solcher Katalysator kann beispielsweise analog der WO 97/34694 hergestellt werden.

Um $C_8$-Diole in Anwesenheit von Wasserstoff mit einem Katalysator in Kontakt zu bringen, werden $C_8$-Diole und Wasserstoff in einen Reaktor eingeleitet, in diesem sich der Katalysator befindet.

$C_8$-Diole werden in der flüssigen Phase in den Reaktor eingeleitet. Es ist bevorzugt, dass die $C_8$-Diole mit einem flüssigen Inertmedium verdünnt sind. Dies hat beispielsweise den Vorteil, dass die Viskosität der $C_8$-Diole erniedrigt werden kann, wodurch diese leichter förderbar sind. Auch kann die Verdünnung der $C_8$-Diole mit einem flüssigen Inertmedium dazu dienen, die Wärmeabführung während der Reaktion günstig zu beeinflussen.

Ein Inertmedium geht unter den Reaktionsbedingungen keine Reaktion mit den Edukten, Produkten und/oder Katalysator ein. Beispielsweise handelt es sich bei einem flüssigen Inertmedium um einen langkettigen Kohlenwasserstoff. Der Anteil an einem Inertmedium beträgt bevorzugt 10 bis 80 Gewichtsprozent, weiter bevorzugt 20 bis 70 Gewichtsprozent und besonders bevorzugt 25 bis 65 Gewichtsprozent, bezogen auf das Gesamtgewicht von $C_8$-Diolen und Inertmedium.

Es ist bevorzugt, dass die $C_8$-Diole so mit dem Katalysator in Kontakt gebracht werden, dass sich eine Gesamtflüssigkeitsbelastung des Katalysators von 0,01 bis 5 $g_{Flüssigphase}/(Ml_{Katalysator}*h)$ ergibt. Es ist weiter bevorzugt, dass sich eine Gesamtflüssigkeitsbelastung von 0,3 bis 5 $g_{Flüssig-phase}/(ml_{Katalysator}*h)$ ergibt. Werden die $C_8$-Diole mit einem flüssigen Inertmedium verdünnt, ist darauf zu achten, dass sich die Gesamtflüssigkeitsbelastung des Katalysators in dem zuvor definierten Rahmen bewegt.

Wasserstoff wird bevorzugt möglichst rein in den Reaktor eingeleitet. Reiner Wasserstoff hat eine Reinheit von mindestens 95 Gewichtsprozent. Verunreinigungen im Wasserstoff können beispielsweise Methan und/oder Stickstoff sein.

Wasserstoff wird mindestens in der stöchiometrischen Menge, bezogen auf die hydrogenolytisch zu spaltenden $C_8$-Diole, in den Reaktor eingeleitet. So kann die Menge an Wasserstoff die in den Reaktor eingeleitet wird 100 bis 300 Prozent der stöchiometrischen Menge, bezogen auf die Menge an hydrogenolytisch zu spaltenden $C_8$-Diole, betragen. Es ist bevorzugt, dass die Menge an Wasserstoff 105 bis 200 Prozent, weiter bevorzugt 110 bis 180 Prozent und besonders bevorzugt 120 bis 160 Prozent beträgt. Die Menge an Wasserstoff kann beispielsweise 101, 102, 108, 112, 115, 118, 122, 125, 130, 135, 140, 145, 150 oder 155 Prozent der stöchiometrischen Menge, bezogen auf die Menge an hydrogenolytisch zu spaltenden $C_8$-Diole, betragen.

Es ist zweckmäßig den Katalysator vor Verwendung in dem erfindungsgemäßen Verfahren in eine aktive Form zu überführen. Der Katalysator wird hierzu beispielsweise in Gegenwart eines wasserstoffhaltigen Gases nach einem Temperaturprogramm reduziert. Bei einem wassersstoffhaltigen Gas handelt es sich um ein Gas, das 1 bis 99 Prozent Wasserstoff und 99 bis 1 Prozent eines gasförmigen Inertmediums wie Stickstoff, Argon und/oder Helium enthält. Um beispielsweise eine bessere Wärmeabführung während der Aktivierung des Katalysators zu gewährleisten, ist es weiterhin zweckmäßig, die Aktivierung in Gegenwart eines flüssigen Inertmediums durchzuführen. Bei einem flüssigen Inertmedium handelt es sich beispielsweise um einen langkettigen Kohlenwasserstoff oder um eine Mischung von langkettigen Kohlenwasserstoffen.

Als Reaktor für das erfindungsgemäße Verfahren eigenen sich prinzipiell alle Reaktoren in denen Hydrierreaktionen, vorzugsweise Hydrierreaktionen in der Flüssigphase, durchgeführt werden können und die für eine Hydrierung bei einer Temperatur von 150 bis 300°C und einen Druck von 20 bis 300 bar geeignet sind. So können beispielsweise Rührkesselreaktoren, Rührkesselkaskaden, Kammerreaktoren, Blasensäulen, Schlaufenreaktoren, Rieselfilmreaktoren, Bodenkolonnen, Dünnschichtreaktoren, Strahldüsenreaktoren, pulsierende Kolonnen, Festbettreaktoren, Festbett-Röhrenreaktoren, Festbettreaktoren mit innenliegenden oder außenliegenden Wärmetauschern, Wirbelschichtreaktoren, mehrstufige Wirbelschichtreaktoren, Wanderbettreaktoren, oder eine beliebige Kombination dieser für das erfindungsgemäße Verfahren genutzt werden. Mehrere gleiche oder verschiedene Reaktortypen können in Reihe oder parallel geschaltet sein. Reaktoren, die sich für das erfindungsgemäße Verfahren eignen sind beispielsweise in J. Falbe, Katalysatoren, Tenside und Mineralöladditive, Georg Thieme Verlag, Stuttgart, 1978, Seite 30 bis 37 beschrieben.

Es sind solche Reaktortypen bevorzugt, bei denen der Katalysator in einem Festbett oder in einer Schüttung angeordnet ist. Es ist möglich, dass in einem Reaktor mehrere Katalysator Festbetten oder Schüttungen in Reihe angeordnet sind. Der Reaktor untergliedert sich dabei in Zonen mit Katalysator (Reaktionszonen) und Zonen ohne Katalysator, die sich gegenseitig abwechseln. Die Anzahl der Reaktionszonen in einem Reaktor kann 2 bis 10 betragen.

Die $C_8$-Diole und Wasserstoff werden über eine oder mehrere Zuleitungen in den Reaktor eingeleitet. Es ist bevorzugt, dass die $C_8$-Diole und Wasserstoff über eine oder mehrere separate Zuleitungen in den Reaktor eingeleitet werden. Auch wenn nicht bevorzugt, ist es generell auch möglich, dass die $C_8$-Diole und Wasserstoff vorvermischt werden und über eine oder mehrere gemeinsame Zuleitungen in den Reaktor eingeleitet werden.

Es ist bevorzugt, dass die $C_8$-Diole in Rieselfahrweise oder in Sumpffahrweise in den Reaktor eingeleitet werden. Die $C_8$-Diole werden dabei im Gleich- oder Gegenstrom mit Wasserstoff in den Reaktor eingeleitet, wobei ein Einleiten im Gleichstrom bevorzugt ist.

Wird für das erfindungsgemäße Verfahren ein Reaktor mit mehreren Reaktionszonen verwendet, kann Wasserstoff auch zwischen den Reaktionszonen in den Reaktor eingeleitet und dort mit den $C_8$-Diolen in Kontakt gebracht werden. Dies hat beispielsweise den Vorteil, dass die Temperaturentwicklung während der Reaktion besser kontrolliert werden kann. Eine vorteilhafte Verfahrensausgestaltung ist beispielsweise in der EP-A1 0 073 129 beschrieben.

Das erfindungsgemäße Verfahren wird kontinuierlich oder diskontinuierlich ausgeführt, wobei eine kontinuierliche Ausführung bevorzugt ist.

Das erfindungsgemäße Verfahren kann adiabatisch oder nicht adiabatisch ausgeführt werden. Wird das erfindungsgemäße Verfahren adiabatisch ausgeführt, ist es von Vorteil, wenn ein Reaktor mit mehreren Reaktionszonen verwendet wird. Während die Reaktion in den einzelnen Reaktionszonen weitgehend adiabatisch abläuft, können zwischen den Reaktionszonen kältere Edukte in Form von Wasserstoff oder frischen $C_8$-Diolen zugeleitet werden. Dies ermöglicht eine weitgehend adiabatische Reaktionsführung mit verbesserter Temperaturkontrolle. Eine vorteilhafte Ausgestaltungsform für ein derartiges Verfahren ist beispielsweise in der EP-A1 0 073 129 offenbart.

Im Rahmen der vorliegenden Erfindung werden die $C_8$-Diole, Wasserstoff und der Katalysator im Allgemeinen bei einer Temperatur von 150 bis 300°C, bevorzugt 180 bis 260°C in Kontakt gebracht.

Die $C_8$-Diole, Wasserstoff und der Katalysator werden im Allgemeinen bei einem Druck von 20 bis 300 bar, bevorzugt 100 bis 300 bar und weiter bevorzugt 150 bis 280 bar in Kontakt gebracht.

Es ist bevorzugt, dass die $C_8$-Diole, Wasserstoff und Katalysator bei einer Temperatur von 150 bis 300°C und einem Druck von 100 bis 300 bar und weiter bevorzugt bei einer Temperatur von 180 bis 260°C und einem Druck von 150 bis 280 bar in Kontakt gebracht werden.

Durch das erfindungsgemäße Verfahren wird eine Reaktionsmischung erhalten, die auch als rohes $C_8$-Diol-Hydrierprodukt bezeichnet wird. Das rohe $C_8$-Diol-Hydrierprodukt aus dem erfindungsgemäßen Verfahren, bzw. aus der erfindungsgemäßen Hydrierung, kann neben $C_4$-Monohydroxyverbindungen verschiedene Nebenprodukte, wie hochsiedende Nebenprodukte, enthalten.

Das rohe $C_8$-Diol-Hydrierprodukt wird einer Destillation unterzogen. Bei einer Destillation handelt es sich um eine normale Destillation oder Rektifikation. Die Destillation erfolgt kontinuierlich oder diskontinuierlich. Die Destillation wird in einer oder mehreren Destillationskolonnen ausgeführt. Generell ist es vorteilhaft die Destillation als Rektifikation auszuführen, wobei eine oder mehrere Rektifikationskolonnen verwendet werden. Wird die Rektifikation in mehreren Rektifikationskolonnen ausgeführt ist es vorteilhaft, mehrere hintereinandergeschaltete Rektifikationskolonnen, beispielsweise 2, 3 oder 4, zu verwenden.

Als Kolonnen für die Destillation, beziehungsweise Rektifikation, eignen sich beispielsweise Bodenkolonnen, wie Ventilbodenkolonne. Im Allgemeinen sind Kolonnen mit Packungen bevorzugt. Bei Packungen handelt es sich beispielsweise um regellose Schüttungen oder um geordnete Packungen handeln. Im Allgemeinen sind geordnete Packungen bevorzugt. Die Zahl der Trennstufen kann der Fachmann aufgrund seines Fachwissens und durch wenige Routineversuche an die gewünschte Trennwirkung anpassen.

Vorteilhafte Verfahrensausgestaltungen zur Destillation eines rohen $C_8$-Diol-Hydrierprodukts sind in der EP-B1 1 165 480 offenbart. Obwohl das in der EP-B1 offenbarte Verfahren zur Destillation von Aldehyden beschrieben wird, kann dieses auch zur Destillation von $C_4$-Monohydroxyverbindungen genutzt werden.

Enthält das rohe $C_8$-Diol-Hydrierprodukt noch hochsiedende Nebenprodukte kann es zweckmäßig sein, dieses zumindest teilweise in das erfindungsgemäße Verfahren zurückzuführen, bevor dieses einer Destillation unterzogen wird. Wird das rohe $C_8$-Diol-Hydrierprodukt zumindest teilweise in das erfindungsgemäße Verfahren zurückgeführt, wird das rohe $C_8$-Diol-Hydrierprodukt aufgeteilt, wobei ein Teil dem Verfahren entnommen wird, während der andere Teil in das erfindungsgemäße Verfahren zurückgeführt wird. Das Verhältnis zwischen dem Teil, der dem Verfahren entnommen wird, zu dem Teil, der in das erfindungsgemäße Verfahren zurückgeführt wird, beträgt bevorzugt 1:1 bis 20:1. Es ist bevorzugt, dass das Verhältnis 2:1 bis 10:1 beträgt. Der Teil der in das erfindungsgemäße Verfahren zurückgeführt wird kann mit frischen $C_8$-Diolen vermischt werden oder separat dem erfindungsgemäßen Verfahren zugeleitet werden. Das Verhältnis zwischen frischen $C_8$-Diolen zu dem Teil der in das erfindungsgemäße Verfahren zurückgeführt wird beträgt bevorzugt 1:1 bis 1:20. Es ist weiter bevorzugt, dass das Verhältnis 1:4 bis 1:15 beträgt. Es ist besonders bevorzugt, dass das Verhältnis 1:4 zu 1:10 beträgt.

Die Rückführung von zumindest einem Teil des rohen $C_8$-Diol-Hydrierprodukts in das erfindungsgemäße Verfahren erlaubt einen wirtschaftlichen Betrieb des Verfahrens auch bei niedrigeren Umsätzen. Durch niedrigere Umsätze kann die Verweilzeit und damit die thermische Belastung der $C_8$-Diole reduziert werden. Diese neigen daher weniger zur Bildung von unerwünschten Nebenprodukten, die zur Verkokung des Katalysators führen können.

Wird ein Teil in das erfindungsgemäße Verfahren zurückgeführt ist darauf zu achten, dass sich die Gesamtflüssigkeitsbelastung des Katalysators in dem zuvor definierten Rahmen bewegt.

Der Teil der dem erfindungsgemäßen Verfahren entnommen wird, wird einer Destillation unterzogen.

Gegenstand der vorliegenden Erfindung ist demnach auch ein Verfahren zur Herstellung von n-Butanol, iso-Butanol oder einer Mischung dieser durch Hydrogenolyse von $C_8$-Diolen, das dadurch gekennzeichnet ist, dass $C_8$-Diole in Anwesenheit eines Katalysators bei einer Temperatur von 150 bis 300°C, vorzugsweise 180 bis 260°, und einem Druck von 100 bis 300 bar, vorzugsweise 150 bis 280 bar in Kontakt gebracht werden und die $C_8$-Diole zumindest teilweise zu n-Butanol, iso-Butanol oder einer Mischung dieser umgesetzt werden und das erhaltene rohe $C_8$-Diol-Hydrierprodukt einer Destillation unterzogen wird, wobei der Katalysator 40 bis 80 Gewichtsprozent Kupferoxid (CuO), 5 bis 60 Gewichtsprozent Aluminiumoxid und 0 bis 30 Gewichtsprozent Manganoxid (MnO), Lanthanoxid oder Zinkoxid bezogen auf das Gesamtgewicht des im Katalysator enthaltenen oxidischen Materials nach Calcinierung, enthält, die

Summe der Gewichtsanteile sich zu 90 bis 100 Prozent addiert und der Anteil an oxidischem Material 80 bis 100 Gewichtsprozent bezogen auf das Gesamtgewicht des Katalysators nach Calcinierung beträgt.

Ebenfalls Gegenstand der vorliegenden Erfindung ist die Verwendung der zuvor offenbarten Katalysatoren zur Herstellung von $C_4$- bis $C_{13}$-Monohydroxyverbindungen.

Demnach ist hier beschrieben die Verwendung der zuvor offenbarten Katalysatoren in dem erfindungsgemäßen Verfahren zur Herstellung von $C_4$- bis $C_{13}$-Monohydroxyverbindungen aus einer Sumpffraktion in Anwesenheit von Wasserstoff, wobei die Sumpffraktion bei der Destillation eines Rohgemisches von $C_4$- bis $C_{13}$-Oxoaldehyden aus dem Austrag der kobalt- oder rhodiumkatalysierten Hydroformylierung oder bei der Destillation eines Rohgemisches von $C_4$- bis $C_{13}$-Oxoalkoholen anfällt,

oder

die Verwendung der zuvor offenbarten Katalysatoren in dem erfindungsgemäßen Verfahren zur Herstellung von n-Butanol und/oder iso-Butanol aus einer Sumpffraktion in Anwesenheit von Wasserstoff, wobei die Sumpffraktion bei der Destillation eines Rohgemisches von $C_4$-Oxoaldeyhden aus der rhodiumkatalysierten Hydroformylierung oder bei der Destillation eines Rohgemisches von $C_4$-Oxoalkoholen, anfällt,

oder

die Verwendung der zuvor offenbarten Katalysatoren in dem erfindungsgemäßen Verfahren zur Herstellung von n-Butanol und/oder iso-Butanol aus einer Sumpffraktion in Anwesenheit von Wasserstoff, wobei die Sumpffraktion bei der Destillation eines Rohgemisches von $C_4$-Oxoaldeyhden aus der rhodiumkatalysierten Hydroformylierung oder bei der Destillation eines Rohgemisches von $C_4$-Oxoalkoholen anfällt und die Herstellung bei einer Temperatur von 180 bis 260 °C und einem Druck von 150 bis 280 bar erfolgt.

Auch hier beschrieben ist die Verwendung der zuvor offenbarten Katalysatoren in dem erfindungsgemäßen Verfahren zur Herstellung von $C_4$-Monohydroxyverbindungen durch Hydrogenolyse von $C_8$-Diolen,

oder

die Verwendung der zuvor offenbarten Katalysatoren in dem erfindungsgemäßen Verfahren zur Herstellung von n-Butanol, iso-Butanol oder einer Mischung dieser durch Hydrogenolyse von $C_8$-Diolen, wobei die $C_8$-Diole in einer Sumpffraktion enthalten sind, die bei der Destillation eines Rohgemisches von $C_4$-Oxoaldehyden aus dem Austrag der rhodiumkatalysierten Hydroformylierung, oder bei der Destillation eines Rohgemisches von $C_4$-Oxoalkoholen anfällt,

oder

die Verwendung der zuvor offenbarten Katalysatoren in dem erfindungsgemäßen Verfahren zur Herstellung von n-Butanol und/oder iso-Butanol durch Hydrogenoloyse von $C_8$-Diolen, wobei die $C_8$-Diole in einer Sumpffraktion enthalten sind, die bei der Destillation eines Rohgemisches von $C_4$-Oxoaldehyden aus dem Austrag der rhodiumkatalysierten Hydroformylierung, oder bei der Destillation eines Rohgemisches von $C_4$-Oxoalkoholen anfällt und die Herstellung bei einer Temperatur von 180 bis 260 °C und einem Druck von 150 bis 280 bar erfolgt,

Gegenstand der Erfindung ist auch die Verwendung einer Sumpffraktion zur Herstellung von $C_4$-bis $C_{13}$-Monohydroxyverbindungen nach dem erfindungsgemäßen Verfahren. Die Sumpffraktion fällt bei der Destillation eines Rohgemisches von $C_4$- bis $C_{13}$-Oxoaldehyden aus dem Austrag der kobalt- oder rhodiumkatalysierten Hydroformylierung oder bei der Destillation eines Rohgemisches von $C_4$- bis $C_{13}$-Oxoalkoholen an. Es ist bevorzugt, dass es sich um ein Rohgemisch von $C_4$- bis $C_9$-Oxoaldehyden oder $C_4$- bis $C_9$-Oxoalkoholen handelt. Weiter ist es bevorzugt, dass es sich um ein Rohgemisch von $C_4$-oder $C_9$-Oxoaldehyden oder $C_4$- oder $C_9$-Oxoalkoholen handelt. Insbesondere ist es bevorzugt, dass es sich um ein Rohgemisch von $C_4$-Oxoaldeyhden, wie n- und/oder iso-Butanal, oder $C_4$-Oxoalkoholen, wie n- und/oder iso-Butanol handelt.

Isoheptanol, Isooctanol, Isononanol, Isodecanol, Isoundecanol oder Isotridicanol, sowie die entsprechenden Aldehyde, die sich aus den vorgenannten Alkoholen ableiten, stellen keine chemischen Einzelverbindungen sondern Isomerenmischungen dar. Die Zusammensetzung der Isomerenmischungen hängt im Allgemeinen von den zur Herstellung eingesetzten Ausgangsverbindungen und/oder den Herstellbedingungen ab. Abhängig von den zur Herstellung eingesetzten Ausgangsverbindungen und/oder Herstellungsbedingungen sind in der WO2015/082676 beispielhaft Zusammensetzungen von Isomerenmischungen offenbart.

## Beispiele

Die Beispiele sollen der Veranschaulichung der vorliegenden Erfindung dienen und keinen einschränkten Charakter auf diese haben.

In allen Beispielen wurde Wasserstoff in einem Überschuss von 120 bis 160 %, bezogen auf die Menge, die stöchiometrisch zur Hydrierung bzw. Hydrogenolyse der Edukte benötigt wird, eingesetzt.

Die Bestimmung des Kalium- und Phosphorgehalts erfolgte per Elementaranalyse (Atomabsorptionsspektroskopie).

Die Bestimmung der Esterzahl erfolgte analog EN ISO 3681.

Die Bestimmung der Säurezahl erfolgte analog EN ISO 2114.

Die Berechnung der Ausbeute an $C_4$- bis $C_{13}$-Monohydroxyverbindungen, die durch das erfindungsgemäße Verfahren aus einer Sumpffraktion erhalten wird, soll im Folgenden beispielhaft für die $C_4$-Monohydroverbindungen, n-Butanol und iso-Butanol dargelegt werden.

In einem ersten Schritt wird die Sumpffraktion, die dem erfindungsgemäßen Verfahren zugeführt wird, dahingehend analysiert, ob die darin enthaltenen Komponenten formal auf $C_4$- oder $C_8$-Bestandteile zurückgeführt werden können. Die Analyse erfolgt mittels GC nachdem die Retentionszeiten der einzelnen Verbindungen durch GC/MS aufgeklärt wurden.

Die in der Sumpffraktion enthaltenen Komponenten werden dann wie folgt eingeteilt:

Iso-Butanol: 100% $C_4$
n-Butanol: 100% $C_4$
n-Butyl-Butyrat: 100% $C_4$
N-Butyraldehyd-n,n-Diacetal: 100% $C_4$
$C_8$-Diole: 100% $C_8$
2-Ethylhexan-1-ol: 100% $C_8$
$C_{12}$-Ester: 33% $C_4$, 67% $C_8$

So werden beispielsweise iso-Butanol, n-Butanol, n-Butyl-Butyrat, n-Butyraldehyd-n,n-diacetat formal zu 100 % auf $C_4$-Bestandteile zurückgeführt. $C_8$-Diole und 2-Ethylhexan-1-ol werden beispielsweise formal zu 100% auf $C_8$-Bestandteile zurückgeführt. $C_{12}$-Ester werden beispielsweise formal zu 33% auf $C_4$ und zu 67% auf $C_8$-Bestandteile zurückgeführt.

Durch die Summierung der Komponenten kann ein $C_4$-Anteil, ein $C_8$-Anteil und ein Anteil an nicht aufgeschlüsselten Komponenten im Zulauf bestimmt werden. Die Summe an n-Butanol und iso-Butanol, die im Austrag erhalten wird ist die $C_4$-Gesamtausbeute.

Unter der Annahme, dass der Umsatz von den $C_4$-Komponenten im Zulauf zu n-Butanol und iso-Butanol vollständig abläuft, kann anhand des Wertes der $C_4$-Gesamtausbeute im Austrag die Umsetzung der $C_8$-Komponenten wie folgt berechnet werden:

$C_4$-Gesamtausbeute – $C_4$-Anteil im Zulauf – Anteil nicht aufgeschlüsselter Komponenten im Zulauf = Ausbeute durch Umsetzung von $C_8$-Komponenten zu $C_4$-Komponenten

Durch diese Berechnungsmethode kann festgestellt werden, wie viel $C_8$-Komponenten zu n-Butanol und iso-Butanol umgesetzt werden. Die Bestimmung der Zusammensetzung des Austrags erfolgt ebenfalls mittels GC nachdem die Retentionszeiten der einzelnen Verbindungen durch GC/MC aufgeklärt wurden.

Die Prozentangaben der einzelnen Komponenten beziehen sich auf GC-Flächenprozent.

Beispiel 1:

Der Austrag der rhodiumkatalysierten Hydroformylierung wurde per Rektifikation in n-Butyraldehyd und iso-Butyraldehyd aufgetrennt. Der so erhaltene n-Butyraldehyd wurde einer Hydrierung zugeführt und der Austrag der Hydrierung durch Rektifikation aufgetrennt. Die bei dieser Rektifikation angefallene Sumpffraktion wurde als Edukt verwendet.

Zusammensetzung Edukt:

3% n-Butanol
3% n-Butyl-n-Butyrat
9% n-Butyaldehyd-n,n-Diacetal
47% $C_8$-Diole
12% Ethylhexan-1-ol
21% $C_{12}$-Ester
5% Sonstiges
500 mg/kg Kalium
100 mg/kg Phosphor
0,01% Wasser

Esterzahl Edukt: 76 mgKOH/g.

$C_4$-Anteil Zulauf: 22%
$C_8$-Anteil Zulauf: 73%.

Das Edukt wurde mit Wasserstoff im Überschuss vermischt und in Rieselfahrweise über einen mit einem Katalysator, der 24 Gew.-% Aluminiumoxid, 72 Gew.-% Kupferoxid (CuO) und 4 Gew.-% Lanthanoxid, bezogen auf das Gesamtgewicht des Katalysators nach Calcinierung enthält, befüllten Reaktor bei 240°C, einem Druck von 200 bar und einer Katalysatorbeladung von 0,32 $g_{Edukt}$ / ($ml_{Katalysator}$ x h) geführt.

Zusammensetzung Austrag:

44% n-Butanol
10% i-Butanol
14% 2-Ethylhexan-1-ol
32% Sonstiges
220 mg/kg Kalium
28 mg/kg Phosphor

Esterzahl Austrag: 4mgKOH/g.

$C_4$-Gesamtausbeute: 54%.
Ausbeute durch Umsetzung von $C_8$-Komponenten zu $C_4$-Komponenten: 27%.
Prozentualer Umsatz von $C_8$- zu $C_4$-Komponenten: 37%.

Beispiel 2:

Der Austrag der rhodiumkatalysierten Hydroformylierung von Propen wurde per Rektifikation in n-Butyraldehyd und iso-Butyraldehyd aufgetrennt. Der so erhaltene n-Butyraldehyd wurde einer Hydrierung zugeführt und der Austrag der Hydrierung durch Rektifikation aufgetrennt. Die bei dieser Rektifikation angefallene Sumpffraktion wurde als Edukt verwendet. Im Vergleich zu Beispiel 1 wurde das Edukt zusätzlich mit 3x 20 Gew.% demineralisiertem Wasser, bezogen auf das Gewicht des Edukts, extrahiert. Die organische Phase wurde mit einem Überschuss an Wasserstoff vermischt und in Rieselfahrweise über einen mit einem Katalysator, der 24 Gew.-% Aluminiumoxid, 72 Gew.-% Kupferoxid (CuO) und 4 Gew.-% Lanthanoxid, bezogen auf das Gesamtgewicht des Katalysators nach Calcinierung enthält, befüllten Reaktor bei 240°C, einem Druck von 200 bar und einer Katalysatorbeladung von 0,32 $g_{Edukt}$ / ($ml_{Katalysator}$ x h) geführt.

Zusammensetzung Austrag:

49% n-Butanol
11% iso-Butanol
16% 2-Ethylhexanol
24% Sonstiges
130 mg/kg Kalium
28 mg/kg Phosphor

Esterzahl Austrag: 7mgKOH/g.

$C_4$-Gesamtausbeute: 60%.
Ausbeute durch Umsetzung von $C_8$-Komponenten zu $C_4$-Komponenten: 33%.
Prozentualer Umsatz von $C_8$- zu $C_4$-Komponenten: 45%.

Durch Extraktion der Sumpffraktion mit Wasser kann der Kalium-Anteil im Austrag reduziert und die Gesamtausbeute und damit die Umsetzung von $C_8$-Komponenten zu $C_4$-Komponenten erhöht werden.

Beispiel 3:

Der Austrag der rhodiumkatalysierten Hydroformylierung von Propen wurde per Rektifikation in n-Butyraldehyd und iso-Butyraldehyd aufgetrennt. Der so erhaltene n-Butyraldehyd wurde einer Hydrierung zugeführt und der Austrag der Hydrierung durch Rektifikation aufgetrennt. Die bei dieser Rektifikation angefallene Sumpffraktion wurde als Edukt verwendet. Das Edukt wurde über einen Fallfilmverdampfer destilliert. Das Destillat wurde gesammelt.

Zusammensetzung Edukt:

4% Butanol
9% n-Butyl-n-Butyrat
1% n-Butyraldehyd-n,n-Dibutylacetat
55% $C_8$-Diole
12% 2-Ethylhexan-1-ol
18% $C_{12}$-Ester
1% Sonstiges
470 mg/kg Kalium
75 mg/kg Phosphor

Esterzahl Edukt :86 mgKOH/g.

Zusammensetzung Destillat:

4% n-Butanol
8% n-Butyl-n-Butyrat
1% n-Butyraldehyd-n,n-Dibutylacetal
55% $C_8$-Diole
12% 2-Ethylhexan-1-ol
18% $C_{12}$-Ester
1% Sonstiges
<3 mg/kg Kalium
84 mh/kg Phosphor

Esterzahl Destillats: 84 mgKOH/g.

Beispiel 4:

Ein wie in Beispiel 3 destilliertes Edukt wurde verwendet.

Zusammensetzung Edukt nach Destillation:

2% n-Butanol
7% n-Butyl-n-Butyrat
2% n-Butyraldehyd.-n,n-Dibutylacetal
58% C8-Diole
13% 2-Ethylhexan-1-ol
18% $C_{12}$-Ester
<1% sonstiges
<1 mg /kg Kalium
5 mg/kg Phosphor

Esterzahl Edukt: 87 mgKOH/g.
$C_4$-Anteil im Edukt :17%.
$C_8$-Anteil im Edukt: 83%.

Das Edukt wurde mit Wasserstoff und einem Teil des Austrags vermischt und in Rieselfahrweise über einen mit einem Katalysator, der 24 Gew.-% Aluminiumoxid, 72 Gew.-% Kupferoxid (CuO) und 4 Gew.-% Lanthanoxid, bezogen auf das Gesamtgewicht des Katalysators nach Calcinierung enthält, befüllten Reaktor bei 240°C, einem Druck von 175 bar und einer Katalysatorbeladung von 0,32 $g_{Edukt}$ / ($ml_{Katalysator}$ x h) geführt. Ein Teil des Austrags wurde mit dem Edukt vermischt, dass sich eine Gesamtflüssigkeitsbelastung des Katalysators von 4,7 $g_{Flüssigkeit}$ / ($ml_{Ka-talysator}$ x h) ergibt. Ein Teil des Austrags wird demnach in einer Kreisfahrweise in den Reaktor zurückgeführt.

Zusammensetzung Austrag:

39% n-Butanol

10% i-Butanol
16% 2-Ethylhexan-1-ol
35% Sonstiges
< 3mg/kg Kalium
4 mg/kg Phosphor

Esterzahl Austrag: 14 mgKOH/g.
C4-Gesamtausbeute: 49%.
Ausbeute durch Umsetzung von $C_8$-Komponenten zu $C_4$-Komponenten: 32%.
Prozentualer Umsatz von $C_8$- zu $C_4$-Komponenten: 39%.

Tabelle 1: Übersicht über die Ergebnisse der Beispiele 1, 2 und 4

| | Edukt | | | Produkt | | |
|---|---|---|---|---|---|---|
| Beispiel | $C_4$-Komponenten | $C_8$-Komponenten | Unbekannt | $C_4$-Komponenten | $C_8$-Komponenten | Unbekannt |
| 1 | 22% | 73% | 5% | 54 % | 14 % | 32% |
| 2 | 22% | 73% | 5% | 60% | 16 % | 24 % |
| 4 | 17% | 83% | 0% | 49% | 16 % | 35% |

Beispiel 5:

Zusammensetzung Edukt:

8% n-Butanol
60% $C_8$-Diole
6% 2-Ethylhexan-1-ol
18% $C_{12}$-Ester
8% sonstiges
215 mg/kg Kalium
13 mg/kg Phosphor

Esterzahl Edukt: 71 mgKOH/g.
Säurezahl Edukt: 7mgKOH/g.

$C_4$-Anteil Edukt :14%.
$C_8$-Anteil Edukt: 78%.

Das Edukt wurde mit Wasserstoff vermischt und in Sumpffahrweise über einen mit einem Katalysator befüllten Reaktor bei 220°C, einem Druck von 175 bar und einer Katalysatorbeladung von 3,3 $g_{Flüssigkeit}$ / ($ml_{Katalysator}$ x h) geführt. Der Eduktstrom wurde dabei mit einem Teilstrom aus dem Austrag in einem Verhältnis von 1:10 vermischt und dem Reaktor zugeführt. Wasserstoff wurde dem kombinierten Strom im Überschuss zugeführt.

Als Katalysatoren wurden eingesetzt:
Die Angaben zu Gewichtsprozent beziehen sich auf das Gesamtgewicht des Katalysators nach Calcinierung.

| Katalysator | Aluminiumoxid Gew. % | Kupferoxid (CuO) Gew. % | Lanthanoxid Gew.% | Zinkoxid Gew. % | Manganoxid (MnO) Gew.% | Schüttdichte |
|---|---|---|---|---|---|---|
| 1 | 30 | 60 | | | 10 | 1,31 g/ml |
| 2 | 24 | 72 | 4 | | | 1,21 g/ml |
| 3 | 51 | 49 | | | | 0,87 g/ml |
| 4 | 5 | 70 | | 25 | | 1,32 g/ml |

In der nachfolgenden Tabelle 2 sind die Zusammensetzungen des jeweiligen Austrags mit dem entsprechenden Katalysator angegeben. Wie aus der Tabelle ersichtlich führt der Einsatz von Katalysator 2 zu anfänglich hohen Butanol-Ausbeuten (54,2 %) und hohem Ester-Umsatz. Die anderen Katalysatoren, insbesondere Katalysator 1, führen zwar zu geringeren Butanol-Ausbeuten, es werden jedoch weniger mittelsiedende Nebenprodukte (Summe von Dibutylether und Sonstiges) gebildet. Die $C_4$-Gesamtausbeute durch Umsetzung von $C_8$-Komponenten ist überwiegend größer 20% und beträgt bis zu 40%. Der relative Umsatz von $C_8$- zu $C_4$-Komponetnen beträgt bis zu 74%.

Anhand der Esterzahl und Säurezahl sind maximale Butanolausbeuten von 19,7%, unter der Annahme der vollständigen Hydrierung der Esterfunktionen von n- und/oder iso-Butylbutyrat und der vollständigen Hydrierung von Buttersäure, möglich.

Tabelle 2: Zusammensetzung des Austrags für verschiedene Katalysatoren

| Katalysator | Laufzeit | Temperatur | n-Butanol | i-Butanol | 2-Ethylhexan-1-ol | Dibutylether | $C_8$-Diole | C12-Ester | Sonstiges | Butanol-Ausbeute | Butanol/Nebenprodukte | Esterzahl (Norm) | Säurezahl (Norm) | C4-Gesamtausbeute durch Umsetzung von C8-Komponenten | %-Umsatz von C8-zu C4-Komponenten | K-Gehalt | P-Gehalt |
|---|---|---|---|---|---|---|---|---|---|---|---|---|---|---|---|---|---|
| | h | °C | % | % | % | % | % | % | % | % | - | mg (KOH)/g | mg (KOH)/g | % | % | ppm | ppm |
| 1 | 170,5 | 200 | 41,1 | 15,8 | 6,9 | 2,6 | 18,2 | 3,0 | 12,4 | 49,0 | 7,2 | 13 | 0 | 34,9 | 71,3% | 140 | 7 |
| | 479,2 | 200 | 30,5 | 11,7 | 7,3 | 2,1 | 30,6 | 4,9 | 12,9 | 34,3 | 5,0 | 19 | 0 | 20,2 | 59,0% | 190 | 8 |
| | 569.2 | 210 | 35.9 | 15.1 | 8,0 | 3,3 | 17.4 | 3.7 | 16.6 | 43,0 | 3.7 | 20 | 0 | 29.0 | 67.3% | n.d. | n.d. |
| | 975,45 | 200 | 18,9 | 7,3 | 7,1 | 1,3 | 42,8 | 9,6 | 12,8 | 18,3 | 3,1 | 41 | 0 | 4,2 | 23,1% | n.d. | n.d. |
| 2 | 160,5 | 200 | 43,3 | 18,9 | 7,4 | 3,3 | 9,6 | 0,5 | 17,0 | 54,2 | 4,5 | 0 | 0 | 40,2 | 74,1% | 85 | 7 |
| | 497.5 | 200 | 34.4 | 15.1 | 8.5 | 3.4 | 18.3 | 0.7 | 19.5 | 41,6 | 2.8 | 4 | 0 | 27.5 | 66,2% | 175 | 8 |
| | 574 | 210 | 38,2 | 17,8 | 8,7 | 4,0 | 9,2 | 0,5 | 21,6 | 48,1 | 2,8 | 2 | 0 | 34,0 | 70,8% | n.d. | n.d. |
| | 976 | 200 | 30,4 | 14,0 | 8,0 | 3,4 | 24,3 | 1,5 | 18,4 | 36,5 | 2,7 | 10 | 0 | 22,5 | 61,5% | n.d. | n.d. |
| 3 | 160,5 | 200 | 31,5 | 13,5 | 9,4 | 2,2 | 18,7 | 1,7 | 23,0 | 37,1 | 2,2 | 6 | 0 | 23,0 | 62,1% | 18 | 5 |
| | 497.5 | 200 | 30.5 | 13,2 | 9,0 | 3.1 | 21.7 | 2.0 | 20.5 | 35,8 | 2.3 | 8 | 0 | 21.8 | 60.8% | 160 | 6 |
| | 574 | 210 | 34,4 | 16,1 | 9,4 | 3,9 | 11,4 | 1,2 | 23,5 | 42,6 | 2,2 | 7 | 0 | 28,6 | 67.0% | n.d. | n.d. |
| | 970,5 | 200 | 27,8 | 13,1 | 8,4 | 3,3 | 25,6 | 2,5 | 19,3 | 32,9 | 2,3 | 13 | 0 | 18,9 | 57,4% | n.d. | n.d. |
| 4 | 160,5 | 200 | 30,6 | 13,7 | 10,3 | 3,7 | 14,2 | 0,5 | 26,9 | 36,4 | 1,6 | 0 | 0 | 22,3 | 61,4% | 150 | 7 |
| | 497.5 | 200 | 29.1 | 12.6 | 10.0 | 3,8 | 18.8 | 0,8 | 24.8 | 33,8 | 1.7 | 5 | 0 | 19.7 | 58.4% | 195 | 7 |
| | 574 | 210 | 31,5 | 14,9 | 10,2 | 4,4 | 10,8 | 0,7 | 27,6 | 38,4 | 1,6 | 3 | 0 | 24,4 | 63,4% | n.d. | n.d. |
| | 976 | 200 | 25,6 | 11,9 | 9,2 | 3,9 | 24,3 | 1,6 | 23,6 | 29,5 | 1,5 | 10 | 0 | 15,5 | 52,4% | n.d. | n.d. |
| Feed | - | - | 7,9 | 0,0 | 5,5 | 0,0 | 59,9 | 18,4 | 8,2 | - | - | 71 | 7 | - | - | 215 | 13 |

Im Zulauf (Feed) sind bereits ca. 8% Butanole enthalten. Diese werden von der Summe der n- und iso-Butanole abgezogen, um die Menge an n- und Iso-Butanolen zu bestimmen, die während der Reaktion neu gebildet wurden.

Beispiel 8:

2 g eines Katalysators, der 24 Gew.-% Aluminiumoxid, 72 Gew.-% Kupferoxid und 4 Gew.-% Lanthanoxid, bezogen auf das Gesamtgewicht des Katalysators nach Calcinierung enthält, wurde in einen Autoklaven gefüllt und unter Wasserstoffdruck aktiviert. Anschließend wurden 100 g 2-Ethylhexan-1,3-diol zugegeben und bei 220 °C unter 175 bar Wasserstoffdruck für 8 Stunden gehalten. Von der Flüssigphase des Reaktors wurden Proben gezogen und gaschromatographisch untersucht. Dabei wurden die folgenden Ergebnisse erhalten:

| Zeit in Stunden | Ausbeute 2-Ethylhexan-1,3-diol in % | Ausbeute n-Butanol in % |
|---|---|---|
| 0 | 100 | 0 |
| 2 | 62 | 17 |
| 5 | 27 | 32 |
| 8 | 12 | 38 |

Beispiel 9:

Der Austrag der cobaltkatalysierten Hydroformylierung von Isoocten wurde nach Abtrennung des Hydroformylierungskatalysators einer Hydrierung zugeführt und der Austrag der Hydrierung durch Rektifikation aufgetrennt. Die bei dieser Rektifikation angefallene Sumpffraktion wurde als Edukt verwendet. Das Edukt wurde wie folgt analysiert:

1% Isononanol
99% Hochsieder

Esterzahl Edukt: 55 mgKOH/g.
Säurezahl Edukt: 12mgKOH/g.

Das Edukt wurde mit Wasserstoff im Überschuss vermischt und in Rieselfahrweise über einen mit einem Katalysator, der 24 Gew.-% Aluminiumoxid, 72 Gew.-% Kupferoxid (CuO) und 4 Gew.-% Lanthanoxid, bezogen auf das Gesamtgewicht des Katalysators nach Calcinierung enthält, befüllten Reaktor bei 200°C, einem Druck von 200 bar und einer Katalysatorbeladung von 0,3 $g_{Edukt}$ / ($ml_{Katalysator}$ x h) geführt.

Der Austrag wurde wie folgt analysiert:

32% Isononanol

Esterzahl Austrag: 17mgKOH/g.
Säurezahl kleiner 1mgKOH/g.

Durch die Hydrierung der Säure wurden etwa 3,5 % Isononanol gebildet und durch die Hydrierung des Esters 19 % Isononanol. Es wurden in Summe jedoch 31% Isononanol gebildet, womit die Umwandlung anderer Hochsieder angezeigt wird.

**Patentansprüche**

**1.** Verfahren zur Herstellung von $C_4$- bis $C_{13}$-Monohydroxyverbindungen aus einer Sumpffraktion, die bei der Destillation eines Rohgemisches von $C_4$- bis $C_{13}$-Oxoaldehyden aus der kobalt- oder rhodiumkatalysierten Hydroformylierung, oder bei der Destillation eines Rohgemisches von $C_4$- bis $C_{13}$-Oxoalkoholen, anfällt, **dadurch gekennzeichnet, dass** die Sumpffraktion in Anwesenheit von Wasserstoff mit einem Katalysator, enthaltend Kupferoxid (CuO) und Aluminiumoxid, bei einer Temperatur von 180°C bis 260°C und einem Druck von 150 bar bis 280 bar in Kontakt gebracht wird, das erhaltene rohe Hydrierprodukt einer Destillation unterzogen wird und dass die Menge an $C_4$- bis $C_{13}$-Monohydroxyverbindungen, die nach der Hydrierung im rohen Hydrierprodukt enthalten ist, größer ist als die Menge an $C_4$- bis $C_{13}$-Monohydroxyverbindungen, die sich aus der Hydrierung der in der Sumpffraktion

enthaltenen Ester- und Aldehydverbindungen stöchiometrisch ergibt, einschließlich der in der Sumpffraktion vor der Hydrierung noch enthaltenen $C_4$- bis $C_{13}$-Monohydroxyverbindungen.

**2.** Verfahren gemäß Anspruch 1, wobei der Katalysator Kupferoxid (CuO) mit einem Anteil von 40 bis 80 Gewichtsprozent, Aluminiumoxid mit einem Anteil von 5 bis 60 Gewichtsprozent und 0 bis 30 Gewichtsprozent, Manganoxid (MnO), Lanthanoxid oder Zinkoxid enthält, die Gewichtsprozentangaben sich auf das Gesamtgewicht, des im Katalysator enthaltenen oxidischen Materials nach Calcinierung beziehen, die Summe der Gewichtsanteile sich zu 90 bis 100 Prozent addiert und der Anteil an oxidischem Material im Katalysator mindestens 80 Gewichtsprozent, bezogen auf das Gesamtgewicht des Katalysators nach Calcinierung beträgt.

**3.** Verfahren gemäß einem der Ansprüche 1 bis 2, zur Herstellung von $C_4$- bis Cg-Monohydroxyverbindungen aus einer Sumpffraktion, die bei der Destillation eines Rohgemisches von $C_4$- bis $C_9$-Oxoaldehyden aus der kobalt- oder rhodiumkatalysierten Hydroformylierung, oder bei der Destillation eines Rohgemisches von $C_4$- bis Cg-Oxoalkoholen anfällt.

**4.** Verfahren gemäß Anspruch 3 zur Herstellung von n- und/oder iso-Butanol aus einer Sumpffraktion, die bei der Destillation eines Rohgemisches von $C_4$-Oxoaldehyden aus der rhodiumkatalysierten Hydroformylierung, oder bei der Destillation eines Rohgemisches von $C_4$-Oxoalkoholen anfällt.

**5.** Verfahren gemäß einem der Ansprüche 1 bis 4, wobei die Sumpffraktion vordem in Kontakt bringen mit Wasserstoff und einem Katalysator einer Extraktion, Destillation oder Strippung unterzogen wird.

**6.** Verfahren gemäß einem der Ansprüche 1 bis 4, wobei die Sumpffraktion vordem in Kontakt bringen mit Wasserstoff und einem Katalysator einer Extraktion und Destillation unterzogen wird.

**7.** Verfahren gemäß einem der Ansprüche 1 bis 6, wobei ein Teil des aus der Hydrierung erhaltenen rohen Hydrierprodukts in das offenbarte Verfahren zurückgeführt wird und ein Teil des aus der Hydrierung erhaltenen rohen Hydrierprodukts einer Destillation unterzogen wird.

**8.** Verfahren zur Herstellung von $C_4$-Monohydroxyverbindungen durch Hydrogenolyse von $C_8$-Diolen, **dadurch gekennzeichnet, dass** $C_8$-Diole in Anwesenheit von Wasserstoff mit einem Katalysator enthaltend Kupferoxid und Aluminiumoxid, bei einer Temperatur von 150°C bis 300°C und einem Druck von 20 bar bis 300 bar in Kontakt gebracht werden, die $C_8$-Diole zumindest teilweise zu $C_4$-Monohydroxyverbindungen umgesetzt werden und das erhaltene rohe $C_8$-Diol Hydrierprodukt einer Destillation unterzogen wird.

**9.** Verfahren zur Herstellung von $C_4$-Monohydroxyverbindungen aus der Sumpffraktion, die bei der Destillation eines Rohgemisches von $C_4$-Oxoaldehyden aus der rhodiumkatalysierten Hydroformylierung oder bei der Destillation eines Rohgemisches von $C_4$-Oxoalkoholen anfällt, **dadurch gekennzeichnet,**
**dass** die Sumpffraktion in Anwesenheit von Wasserstoff mit einem Katalysator, enthaltend Kupferoxid und Aluminiumoxid, bei einer Temperatur von 150°C bis 300°C und einem Druck von 20 bar bis 300 bar in Kontakt gebracht wird, das erhaltene rohe Hydrierprodukt einer Destillation unterzogen wird und dass die Menge an n-Butanol, iso-Butanol oder einer Mischung dieser, die nach der Hydrierung im rohen Hydrierprodukt enthalten ist, größer ist als die Menge an n-Butanol, iso-Butanol oder einer Mischung dieser, die sich aus der Hydrierung der in der Sumpffraktion enthaltenen Ester- und Aldehydverbindungen stöchiometrisch ergibt, einschließlich der in der Sumpffraktion vor der Hydrierung noch enthaltenen $C_4$-Monohydroxyverbindungen.

## Claims

**1.** A process for preparing $C_4$ to $C_{13}$ monohydroxy compounds from a bottom fraction arising in the distillation of a crude mixture of $C_4$ to $C_{13}$ oxo-process aldehydes from cobalt-catalyzed or rhodium-catalyzed hydroformylation, or in the distillation of a crude mixture of $C_4$ to $C_{13}$ oxo-process alcohols, which comprises contacting the bottom fraction in the presence of hydrogen with a catalyst comprising copper oxide (CuO) and aluminum oxide, at a temperature of 180°C to 260°C and a pressure of 150 bar to 280 bar and subjecting the resulting crude hydrogenation product to distillation, and the amount of $C_4$ to $C_{13}$ monohydroxy compounds present in the crude hydrogenation product after the hydrogenation being greater than the amount of $C_4$ to $C_{13}$ monohydroxy compounds given stoichiometrically from the hydrogenation of the ester and aldehyde compounds present in the bottom fraction, including the $C_4$ to $C_{13}$ monohydroxy compounds still present in the bottom fraction before the hydrogenation.

2. The process according to claim 1, wherein the catalyst comprises copper oxide (CuO) with a fraction of 40 to 80 weight percent, aluminum oxide with a fraction of 5 to 60 weight percent, and 0 to 30 weight percent of manganese oxide (MnO), lanthanum oxide or zinc oxide, the weight percentages are based on the total weight of the oxidic material present in the catalyst after calcining, the sum of the weight fractions adds up to 90 to 100 percent, and the fraction of oxidic material in the catalyst is at least 80 weight percent, based on the total weight of the catalyst after calcining.

3. The process according to either of claims 1 and 2, for producing $C_4$ to $C_9$ monohydroxy compounds from a bottom fraction arising in the distillation of a crude mixture of $C_4$ to $C_9$ oxo-process aldehydes from cobalt-catalyzed or rhodium-catalyzed hydroformylation, or in the distillation of a crude mixture of $C_4$ to $C_9$ oxo-process alcohols.

4. The process according to claim 3 for producing n-butanol and/or isobutanol from a bottom fraction arising in the distillation of a crude mixture of $C_4$ oxo-process aldehydes from rhodium-catalyzed hydroformylation, or in the distillation of a crude mixture of $C_4$ oxo-process alcohols.

5. The process according to any of claims 1 to 4, wherein the bottom fraction, before being contacted with hydrogen and a catalyst, is subjected to extraction, distillation or stripping.

6. The process according to any of claims 1 to 4, wherein the bottom fraction, before being contacted with hydrogen and a catalyst, is subjected to extraction and distillation.

7. The process according to any of claims 1 to 6, wherein a part of the crude hydrogenation product obtained from the hydrogenation is returned to the disclosed process and a part of the crude hydrogenation product obtained from the hydrogenation is subjected to distillation.

8. A process for preparing $C_4$ monohydroxy compounds by hydrogenolysis of $C_8$ diols, which comprises contacting $C_8$ diols in the presence of hydrogen with a catalyst comprising copper oxide and aluminum oxide, at a temperature of 150°C to 300°C and a pressure of 20 bar to 300 bar, reacting the $C_8$ diols at least partially to give $C_4$ monohydroxy compounds, and subjecting the resulting crude $C_8$ diol hydrogenation product to distillation.

9. A process for preparing $C_4$ monohydroxy compounds from the bottom fraction arising in the distillation of a crude mixture of $C_4$ oxo-process aldehydes from rhodium-catalyzed hydroformylation or in the distillation of a crude mixture of $C_4$ oxo-process alcohols, which comprises
contacting the bottom fraction in the presence of hydrogen with a catalyst comprising copper oxide and aluminum oxide, at a temperature of 150°C to 300°C and a pressure of 20 bar to 300 bar and subjecting the resulting crude hydrogenation product to distillation, and the amount of n-butanol, isobutanol or a mixture thereof present in the crude hydrogenation product after the hydrogenation being greater than the amount of n-butanol, isobutanol or a mixture thereof given stoichiometrically from the hydrogenation of the ester and aldehyde compounds present in the bottom fraction, including the $C_4$ monohydroxy compounds still present in the bottom fraction before the hydrogenation.

## Revendications

1. Procédé pour la préparation de composés monohydroxy en $C_4$-$C_{13}$ à partir d'une fraction de fond qui est produite lors de la distillation d'un mélange brut d'oxoaldéhydes en $C_4$-$C_{13}$ provenant de l'hydroformylation catalysée par le cobalt ou le rhodium ou lors de la distillation d'un mélange brut d'oxoalcools en $C_4$-$C_{13}$, **caractérisé en ce que** la fraction de fond est mise en contact, en présence d'hydrogène, avec un catalyseur contenant de l'oxyde de cuivre (CuO) et de l'oxyde d'aluminium à une température de 180°C à 260°C et à une pression de 150 bars à 280 bars, le produit d'hydrogénation brut obtenu est soumis à une distillation et **en ce que** la quantité de composés monohydroxy en $C_4$-$C_{13}$ qui est contenue après l'hydrogénation dans le produit d'hydrogénation brut est supérieure à la quantité de composés monohydroxy en $C_4$-$C_{13}$ obtenue stoechiométriquement à partir de l'hydrogénation des composés ester et aldéhyde contenus dans la fraction de fond, y compris les composés monohydroxy en $C_4$-$C_{13}$ encore contenus dans la fraction de fond avant l'hydrogénation.

2. Procédé selon la revendication 1, le catalyseur contenant de l'oxyde de cuivre (CuO) en une proportion de 40 à 80% en poids, de l'oxyde d'aluminium en une proportion de 5 à 60% en poids et 0 à 30% en poids d'oxyde de manganèse (MnO), d'oxyde de lanthane ou d'oxyde de zinc, la somme des indications en % en poids se rapportant

au poids total du matériau oxyde contenu dans le catalyseur après calcination, la somme des proportions en poids valant 90 à 100% et la proportion de matériau oxyde dans le catalyseur représentant au moins 80% en poids, par rapport au poids total du catalyseur après calcination.

**3.** Procédé selon l'une quelconque des revendications 1 à 2, pour la préparation de composés monohydroxy en $C_4$-$C_9$ à partir d'une fraction de fond qui est obtenue lors de la distillation d'un mélange brut d'oxoaldéhydes en $C_4$-$C_9$ provenant de l'hydroformylation catalysée par le cobalt ou le rhodium ou lors de la distillation d'un mélange brut d'oxoalcools en $C_4$-$C_9$.

**4.** Procédé selon la revendication 3, pour la préparation de n-butanol et/ou d'isobutanol à partir d'une fraction de fond qui est obtenue lors de la distillation d'un mélange brut d'oxoaldéhydes en $C_4$ provenant de l'hydroformylation catalysée par le rhodium ou lors de la distillation d'un mélange brut d'oxoalcools en $C_4$.

**5.** Procédé selon l'une quelconque des revendications 1 à 4, la fraction de fond étant soumise à une extraction, à une distillation ou à une rectification avant la mise en contact avec l'hydrogène et un catalyseur.

**6.** Procédé selon l'une quelconque des revendications 1 à 4, la fraction de fond étant soumise à une extraction et à une distillation avant la mise en contact avec l'hydrogène et un catalyseur.

**7.** Procédé selon l'une quelconque des revendications 1 à 6, une partie du produit d'hydrogénation brut obtenu lors de l'hydrogénation étant recyclée dans le procédé divulgué et une partie du produit d'hydrogénation brut obtenu lors de l'hydrogénation étant soumise à une distillation.

**8.** Procédé pour la préparation de composés monohydroxy en $C_4$ par hydrogénolyse de diols en $C_8$, **caractérisé en ce que** des diols en $C_8$ sont mis en contact, en présence d'hydrogène, avec un catalyseur contenant de l'oxyde de cuivre et de l'oxyde d'aluminium à une température de 150°C à 300°C et à une pression de 20 bars à 300 bars, les diols en $C_8$ sont transformés au moins partiellement en composés monohydroxy en $C_4$ et le produit d'hydrogénation diol en $C_8$ brut obtenu est soumis à une distillation.

**9.** Procédé pour la préparation de composés monohydroxy en $C_4$ à partir de la fraction de fond qui est produite lors de la distillation d'un mélange brut d'oxoaldéhydes en $C_4$ provenant de l'hydroformylation catalysée par le rhodium ou lors de la distillation d'un mélange brut d'oxoalcools en $C_4$, **caractérisé en ce que** la fraction de fond est mise en contact, en présence d'hydrogène, avec un catalyseur contenant de l'oxyde de cuivre et de l'oxyde d'aluminium à une température de 150°C à 300°C et à une pression de 20 bars à 300 bars, le produit d'hydrogénation brut obtenu est soumis à une distillation et **en ce que** la quantité de n-butanol, d'isobutanol ou d'un mélange de ceux-ci, qui est contenue après l'hydrogénation dans le produit d'hydrogénation brut est supérieure à la quantité de n-butanol, d'isobutanol ou d'un mélange de ceux-ci qui est obtenue à partir de l'hydrogénation des composés ester et aldéhyde contenus dans la fraction de fond, y compris les composés monohydroxy en $C_4$ encore contenus dans la fraction de fond avant l'hydrogénation.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4148830 A
- WO 0114297 A
- EP 0846095 A
- EP 1255720 A
- EP 1165480 A
- DE 19914260 A
- DE 3228881 A
- DE 628987 A2
- DE 2445303 A
- DE 2628987 A
- WO 01087809 A
- DE 2740216 B
- US 3501537 A
- US 5004845 A
- EP 1673332 B1
- EP 1165480 A1
- EP 1165480 B1
- DE 19914260 A1
- EP 0901982 B1
- WO 2004085356 A
- WO 9734694 A
- EP 0073129 A1
- WO 2015082676 A


### In der Beschreibung aufgeführte Nicht-Patentliteratur


- **FALBE.** New Syntheses with Carbon Monoxide. Springer Berlin, 1980, 162-176
- Ullmann's Encyclopedia of Industrial Chemistry. Wiley-VCH, 2013, 1-4
- Ullmann's Encyclopedia of Industrial Chemistry. Wiley-VCH, 2013
- **J. FALBE.** Katalysatoren, Tenside und Mineralöladditive. Georg Thieme Verlag, 1978, 30-37